(19) Europäisches Patentamt
European Patent Office
Office européen des brevets

(11) **EP 2 599 493 A1**

(12) **EUROPEAN PATENT APPLICATION**
published in accordance with Art. 153(4) EPC

(43) Date of publication:
**05.06.2013 Bulletin 2013/23**

(51) Int Cl.:
*A61K 38/00* (2006.01)   *A23K 1/16* (2006.01)
*A23L 1/305* (2006.01)   *A23L 2/52* (2006.01)
*A61K 38/22* (2006.01)   *A61P 31/04* (2006.01)
*A61P 43/00* (2006.01)

(21) Application number: **11812626.7**

(22) Date of filing: **29.07.2011**

(86) International application number:
**PCT/JP2011/067488**

(87) International publication number:
**WO 2012/015037 (02.02.2012 Gazette 2012/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.07.2010 JP 2010173174**

(71) Applicant: **Morinaga Milk Industry Co., Ltd.**
**Minato-ku**
**Tokyo 108-8384 (JP)**

(72) Inventors:
• **MURATA, Mai**
**Zama-shi**
**Kanagawa 252-8583 (JP)**
• **WAKABAYASHI, Hiroyuki**
**Zama-shi**
**Kanagawa 252-8583 (JP)**
• **YAMAUCHI, Koji**
**Zama-shi**
**Kanagawa 252-8583 (JP)**

(74) Representative: **Dean, John Paul et al**
**Withers & Rogers LLP**
**4 More London Riverside**
**London SE1 2AU (GB)**

(54) **ANTIMICROBIAL ACTIVITY ENHANCING AGENT**

(57)     An antimicrobial activity enhancing agent for lactoferrin or a lactoferrin hydrolysate, which contains a milk protein as an active ingredient and has the following properties, and a method for producing the antimicrobial activity enhancing agent, which comprises (a) the step of contacting raw milk with a cation exchange resin to adsorb proteins in the raw milk to the cation exchange resin, (b) the step of washing the cation exchange resin having undergone the adsorption treatment, and flowing an elution solvent to elute a protein fraction having an isoelectric point of 9.0 to 11.0, and (c) the step of subjecting the eluted protein fraction to membrane filtration using an ultrafiltration membrane having a molecular weight cutoff of 30 kDa to prepare the milk protein having the following properties:

(A) an action of enhancing the antimicrobial activity of lactoferrin or a lactoferrin hydrolysate,
(B) a molecular weight of 30 kDa or less,
(C) an isoelectric point of 9.0 to 11.0.

[Fig. 5]

Retention Time (min)

Angiogenin-2 → (About 17kD)
← Angiogenin-1 (About 15kD)

α   β

SEQUENCE LISTING

<110> Morinaga Milk Industry Co., Ltd.

<120> Enhancer of anti-microorganism activity

<130> FP224-11182

<150> JP2010-173174
<151> 2010-07-30

<160> 8

<170> PatentIn version 3.5

<210> 1
<211> 36
<212> PRT
<213> Bos taurus

<400> 1

```
Val Ser Gln Pro Glu Ala Thr Lys Cys Phe Gln Trp Gln Arg Asn Met
1               5                   10                  15

Arg Lys Val Arg Gly Pro Pro Val Ser Cys Ile Lys Arg Asp Ser Pro
            20                  25                  30
Ile Gln Cys Ile
        35
```

<210> 2
<211> 11
<212> PRT
<213> Bos taurus

<400> 2
```
Gly Arg Arg Arg Arg Ser Val Gln Trp Cys Ala
1               5                   10
```

<210> 3
<211> 25
<212> PRT
<213> Bos taurus

<400> 3

```
Phe Lys Cys Arg Arg Trp Gln Trp Arg Met Lys Lys Leu Gly Ala Pro
1               5                   10                  15
Ser Ile Thr Cys Val Arg Arg Ala Phe
            20                  25
```

<210> 4
<211> 125
<212> PRT
<213> Bos taurus

<400> 4
```
Ala Gln Asp Asp Tyr Arg Tyr Ile His Phe Leu Thr Gln His Tyr Asp
1               5                   10                  15
Ala Lys Pro Lys Gly Arg Asn Asp Glu Tyr Cys Phe Asn Met Met Lys
            20                  25                  30
Asn Arg Arg Leu Thr Arg Pro Cys Lys Asp Arg Asn Thr Phe Ile His
```

```
               35                    40                    45
     Gly Asn Lys Asn Asp Ile Lys Ala Ile Cys Glu Asp Arg Asn Gly Gln
          50                    55                    60
     Pro Tyr Arg Gly Asp Leu Arg Ile Ser Lys Ser Glu Phe Gln Ile Thr
     65                    70                    75                    80
     Ile Cys Lys His Lys Gly Gly Ser Ser Arg Pro Pro Cys Arg Tyr Gly
                    85                    90                    95
     Ala Thr Glu Asp Ser Arg Val Ile Val Val Gly Cys Glu Asn Gly Leu
                    100                   105                   110
     Pro Val His Phe Asp Glu Ser Phe Ile Thr Pro Arg His
                    115                   120                   125
```

<210> 5
<211> 148
<212> PRT
<213> Bos taurus

<400> 5

```
     Met Val Met Val Leu Ser Pro Leu Leu Leu Val Phe Ile Leu Gly Leu
     1               5                   10                    15
     Gly Leu Thr Pro Val Ala Pro Ala Gln Asp Asp Tyr Arg Tyr Ile His
                    20                    25                    30
     Phe Leu Thr Gln His Tyr Asp Ala Lys Pro Lys Gly Arg Asn Asp Glu
                    35                    40                    45
     Tyr Cys Phe Asn Met Met Lys Asn Arg Arg Leu Thr Arg Pro Cys Lys
          50                    55                    60
     Asp Arg Asn Thr Phe Ile His Gly Asn Lys Asn Asp Ile Lys Ala Ile
     65                    70                    75                    80
     Cys Glu Asp Arg Asn Gly Gln Pro Tyr Arg Gly Asp Leu Arg Ile Ser
                    85                    90                    95
     Lys Ser Glu Phe Gln Ile Thr Ile Cys Lys His Lys Gly Gly Ser Ser
                    100                   105                   110
     Arg Pro Pro Cys Arg Tyr Gly Ala Thr Glu Asp Ser Arg Val Ile Val
                    115                   120                   125
     Val Gly Cys Glu Asn Gly Leu Pro Val His Phe Asp Glu Ser Phe Ile
          130                   135                   140
     Thr Pro Arg His
     145
```

<210> 6
<211> 148
<212> PRT
<213> Bos taurus

<400> 6

```
     Met Val Met Val Leu Ser Pro Leu Phe Leu Val Phe Ile Leu Gly Leu
     1               5                   10                    15
     Gly Leu Thr Pro Val Ala Pro Ala Gln Asp Asp Tyr Arg Tyr Ile His
                    20                    25                    30
     Phe Leu Thr Gln His Tyr Asp Ala Lys Pro Lys Gly Arg Asn Asp Glu
                    35                    40                    45
     Tyr Cys Phe Asn Met Met Lys Asn Arg Arg Leu Thr Arg Pro Cys Lys
          50                    55                    60
     Asp Arg Asn Thr Phe Ile His Gly Asn Lys Asn Asp Ile Lys Ala Ile
     65                    70                    75                    80
     Cys Glu Asp Arg Asn Gly Gln Pro Tyr Arg Gly Asp Leu Arg Ile Ser
                    85                    90                    95
     Lys Ser Glu Phe Gln Ile Thr Ile Cys Lys His Lys Gly Gly Ser Ser
                    100                   105                   110
     Arg Pro Pro Cys Arg Tyr Gly Ala Thr Glu Asp Ser Arg Val Ile Val
                    115                   120                   125
     Val Gly Cys Glu Asn Gly Leu Pro Val His Phe Asp Glu Ser Phe Ile
          130                   135                   140
```

```
Thr Pro Arg His
145


<210>   7
<211>   123
<212>   PRT
<213>   Bos taurus

<400>   7
Gln Asn Asp Ala Tyr Arg Gly Phe Leu Arg Lys His Tyr Asp Pro Ser
1               5                   10                  15
Pro Thr Gly His Asp Asp Arg Tyr Cys Asn Thr Met Met Glu Arg Arg
                20                  25                  30
Asn Met Thr Arg Pro Cys Lys Asp Thr Asn Thr Phe Ile His Gly Asn
            35                  40                  45
Ser Asp Asp Ile Arg Ala Val Cys Asp Asp Arg Asn Gly Glu Pro Tyr
        50                  55                  60
Arg Asn Gly Leu Arg Arg Ser Arg Ser Pro Phe Gln Val Thr Thr Cys
65                  70                  75                  80
Arg His Arg Gly Gly Ser Pro Arg Pro Pro Cys Arg Tyr Arg Ala Phe
                85                  90                  95
Arg Ala Asn Arg Val Ile Val Ile Arg Cys Arg Asp Gly Phe Pro Ile
            100                 105                 110
His Leu Glu Glu Asn Phe Ile Pro Pro Arg Pro
            115                 120


<210>   8
<211>   147
<212>   PRT
<213>   Bos taurus

<400>   8
Met Val Met Val Leu Ser Pro Leu Phe Leu Val Phe Met Leu Asp Leu
1               5                   10                  15
Gly Leu Thr Pro Gln Thr Leu Ala Gln Asn Asp Ala Tyr Arg Gly Phe
            20                  25                  30
Leu Arg Lys His Tyr Asp Pro Ser Pro Thr Gly His Asp Asp Arg Tyr
            35                  40                  45
Cys Asn Thr Met Met Glu Arg Arg Asn Met Thr Arg Pro Cys Lys Asp
        50                  55                  60
Thr Asn Thr Phe Ile His Gly Asn Ser Asp Asp Ile Arg Ala Val Cys
65                  70                  75                  80
Asp Asp Arg Asn Gly Glu Pro Tyr Arg Asn Gly Leu Arg Arg Ser Arg
                85                  90                  95
Ser Pro Phe Gln Val Thr Thr Cys Arg His Arg Gly Gly Ser Pro Arg
            100                 105                 110
Pro Pro Cys Arg Tyr Arg Ala Phe Arg Ala Asn Arg Val Ile Val Ile
            115                 120                 125
Arg Cys Arg Asp Gly Phe Pro Ile His Leu Glu Glu Asn Phe Ile Pro
            130                 135                 140
Pro Arg Pro
145
```

**Description**

Technical Field

The present invention relates to an antimicrobial activity enhancing agent for lactoferrin or a lactoferrin hydrolysate containing a milk protein as an active ingredient.

Background Art

Lactoferrin (LF) is an iron-binding protein contained in tear, saliva, peripheral blood, milk and so forth in the living body, and it is known to show an antimicrobial activity against harmful microorganisms such as *Escherichia coli, Candida* yeasts and *Clostridium* bacteria (Non-patent document 1). Further, it is also known to show an antimicrobial activity against *Staphylococcus* and *Enterococcus* bacteria at a concentration of 0.5 to 30 mg/ml (Non-patent document 2).

Further, it is also known a composition containing a lactoferrin hydrolysate for inhibiting adhesion of enteropathogenic *Escherichia coli* to alimentary canal epithelial cells (Patent document 1).

In addition to the above, it is known that Lactoferricin (registered trademark), which is a lactoferrin-derived peptide obtainable from a pepsin digestion product of lactoferrin, has an antimicrobial activity (Non-patent documents 3 and 4). Furthermore, it is investigated a method for enhancing antimicrobial activity of lactoferrin or lactoferrin hydrolysate mentioned above by combining an ingredient other than such lactoferrins.

For example, it is known an antimicrobial agent containing a compound selected from the group consisting of decomposition products of lactoferrins, lactoferrin-related antimicrobial peptides, and arbitrary mixtures of these, and a casein decomposition product as active ingredients (Patent document 2).

Further, it is also known an antimicrobial agent containing a compound selected from the group consisting of decomposition products of lactoferrins, lactoferrin-related antimicrobial peptides, and arbitrary mixtures of these, and a metal-binding protein as active ingredients (Patent document 3). As the metal-binding protein, lactoferrins, transferrin, conalbumin and casein phosphopeptides are described.

Further, it is disclosed an antimicrobial agent containing a decomposed product of lactoferrins and/or an antimicrobial peptide derived from the decomposition product, and lysozyme as active ingredients (Patent document 4).

Further, it is known a composition consisting of an emulsion of an enzymatic decomposition product of lactoferrins, an antimicrobial peptide obtainable from an enzymatic decomposition product of lactoferrins or a chemically synthesized peptide containing a part of amino acid sequence of lactoferrins, and an aliphatic acid (Patent document 5).

In addition, it is known an antimicrobial agent containing a newquinolone antimicrobial agent and a hydrolysate of lactoferrins or an antimicrobial peptide derived from a hydrolysate of lactoferrins as active ingredients (Patent document 6).

Angiogenin that is known to be contained in milk etc. is a protein considered to participate in angiogenesis, but many of its functions remain unknown. It has been reported that some proteins classified into angiogenin show an antimicrobial activity. For example, human angiogenin shows an antimicrobial activity against *Candida albicans* and *Enterococcus faecalis,* mouse angiogenin-1 shows an antimicrobial activity against *Candida albicans* and *Streptococcus pneumoniae,* and mouse angiogenin-4 shows an antimicrobial activity against *Enterococcus faecalis* and *Listeria monocytogenes.* However, angiogenin often do not show antimicrobial activity depending on its type and type of objective microorganism (Non-patent document 5).

As described above, it is known that angiogenins show limitative antimicrobial activities. However, it is not known that a milk protein such as angiogenin has an action of enhancing the antimicrobial activity of lactoferrin or a lactoferrin hydrolysate to provide an antimicrobial activity exceeding the antimicrobial activity of the protein itself.

Prior art references

Patent documents

Patent document 1: Japanese Patent Laid-open No. 11-292789
Patent document 2: Japanese Patent Laid-open No. 5-255109
Patent document 3: Japanese Patent Laid-open No. 5-320067
Patent document 4: Japanese Patent Laid-open No. 6-199698
Patent document 5: Japanese Patent Laid-open No. 9-124504
Patent document 6: Japanese Patent Laid-open No. 11-92375

Non-patent documents

Non-patent document 1: Welsh, J.K. et al., Journal of Pediatrics, Vol. 94, pages 1-9, 1979
Non-patent document 2: Nonnecke, B.J. et al., Journal of Dairy Science, Vol. 67, pages 606-613, 1984
Non-patent document 3: Bellamy, W. et al., Biochimica et Biophysica Acta, Vol. 1121, pages 130-136, 1992
Non-patent document 4: Bellamy, W. et al., Journal of Applied Bacteriology, Vol. 73, pages 472-479, 1992
Non-patent document 5: Hooper, L.V. et al., Nature Immunology, Vol. 4, pages 269-273, 2003

Summary of the Invention

Problems to be solved by the Invention

Lactoferrin and lactoferrin hydrolysate (henceforth also collectively referred to as "lactoferrins") are known from the past to have an antimicrobial activity. And in order to further improve the antimicrobial activity of lactoferrins, methods of combining lactoferrins and various compounds have been investigated.
However, combinations that markedly enhance the antimicrobial effect of lactoferrins are scarcely known.
In particular, there has been desired a method that can enhance the antimicrobial activity of lactoferrins even with an extremely small amount of a substance to be added to lactoferrins.
Furthermore, there has been desired a highly safe antimicrobial agent that show no adverse reactions even if it is ingested on a daily basis.

Means for solving the problem

The inventors of the present invention paid attention to the fact that a protein fraction in the middle of purification obtainable in the production process of lactoferrin markedly enhanced the antimicrobial activity of lactoferrins, and conducted various researches on protein components of the fraction.
Then, they confirmed that, among the aforementioned components, a milk protein that do not have antimicrobial activity or showed a limited antimicrobial spectrum by itself markedly enhanced the antimicrobial activity of lactoferrin or lactoferrin hydrolysate, and accomplished the present invention.
As the first aspect, the present invention solving the aforementioned problem provides an antimicrobial activity enhancing agent for lactoferrin or a lactoferrin hydrolysate, which contains a milk protein as an active ingredient and has the following properties:

(A) an action of enhancing the antimicrobial activity of lactoferrin or a lactoferrin hydrolysate,
(B) a molecular weight of 30 kDa or less,
(C) an isoelectric point of 9.0 to 11.0.

According to a preferred embodiment of the first aspect of the present invention, the milk protein contains lactoferrin or a lactoferrin hydrolysate in an amount of 2.0 mass % or less.
According to another preferred embodiment of the first aspect of the present invention, the milk protein is angiogenin-1 and/or angiogenin-2.
According to a still another preferred embodiment of the first aspect of the present invention, the milk protein per se does not have antimicrobial activity against *Escherichia coli.*
According to a still another preferred embodiment of the first aspect of the present invention, the milk protein has a property that, when the protein is used together with lactoferrin or a lactoferrin hydrolysate, it enhances the antimicrobial activity to 7 times or more of lactoferrin or the lactoferrin hydrolysate against *Escherichia coli.*
The second aspect of the present invention relates to an antimicrobial composition comprising the antimicrobial activity enhancing agent of the first aspect of the present invention, and one or plural kinds of substances selected from the group consisting of lactoferrin and a lactoferrin hydrolysate, wherein mass ratio of the milk protein in the antimicrobial activity enhancing agent to lactoferrin and a lactoferrin hydrolysate is 1/80 or more.
The third aspect of the present invention relates to a drug for antimicrobial use comprising the antimicrobial composition of the second aspect of the present invention.
The fourth aspect of the present invention relates to a food or drink comprising the antimicrobial composition of the second aspect of the present invention.
The fifth aspect of the present invention relates to a feed comprising the antimicrobial composition of the second aspect of the present invention.

The sixth aspect of the present invention relates to use of a milk protein having the following properties for the production of an antimicrobial activity enhancing agent for lactoferrin or a lactoferrin hydrolysate:

(A) an action of enhancing the antimicrobial activity of lactoferrin or a lactoferrin hydrolysate,
(B) a molecular weight of 30 kDa or less,
(C) an isoelectric point of 9.0 to 11.0.

According to a preferred embodiment of the sixth aspect of the present invention, the milk protein contains lactoferrin or a lactoferrin hydrolysate in an amount of 2.0 mass % or less.

According to another preferred embodiment of the sixth aspect of the present invention, the milk protein is angiogenin-1 and/or angiogenin-2.

According to a still another preferred embodiment of the sixth aspect of the present invention, the milk protein per se does not have antimicrobial activity against *Escherichia coli.*

According to a still another preferred embodiment of the sixth aspect of the present invention, the milk protein has a property that, when the protein is used together with lactoferrin or a lactoferrin hydrolysate, it enhances the antimicrobial activity of lactoferrin or the lactoferrin hydrolysate against *Escherichia coli* 7 times or more.

The seventh aspect of the present invention relates to a method for producing an antimicrobial activity enhancing agent for lactoferrin or a lactoferrin hydrolysate comprising the aforementioned milk protein as an active ingredient, which comprises: (a) the step of contacting raw milk with a cation exchange resin to adsorb proteins in the raw milk to the cation exchange resin, (b) the step of washing the cation exchange resin having undergone the aforementioned adsorption treatment, and flowing an elution solvent to elute a protein fraction having an isoelectric point of 9.0 to 11.0, and (c) the step of subjecting the eluted protein fraction to membrane filtration using an ultrafiltration membrane having a molecular weight cutoff of 30 kDa to prepare a milk protein having the following properties:

(A) an action of enhancing the antimicrobial activity of lactoferrin or a lactoferrin hydrolysate,
(B) a molecular weight of 30 kDa or less,
(C) an isoelectric point of 9.0 to 11.0.

Brief Description of the Drawings

Fig. 1 is a drawing (photograph) showing the result of two-dimensional electrophoresis of the antimicrobial activity enhancing agent of the present invention.

Fig. 2 shows a densitometry chart (partially consisting of photograph) of the antimicrobial activity enhancing agent of the present invention.

Fig. 3 shows the primary structure of human Lactoferricin (amino acid sequence: SEQ ID NOS: 1 and 2).

Fig. 4 shows the primary structure of bovine Lactoferricin (amino acid sequence: SEQ ID NO: 3).

Fig. 5 shows an elution curve of the antimicrobial activity enhancing agent of the present invention obtained in gel filtration chromatography (partially consisting of photograph).

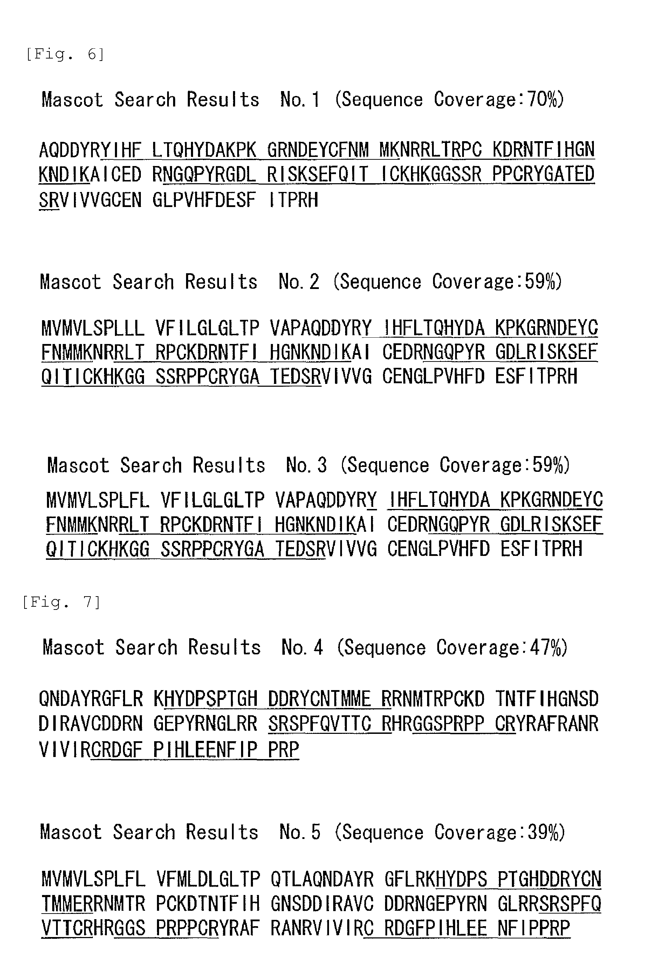

Fig. 6 shows the results of identification of angiogenin-1 obtained by mass spectrometry in which measurement was performed for the same spots obtained in the two-dimensional electrophoresis. The amino acid sequence of bovine angiogenin-1 (SEQ ID NO: 4) is shown as the upper row, the amino acid sequence of bovine angiogenin-1 precursor comprising the amino acid sequence of SEQ ID NO: 4 and additional 23 amino acid residues existing before the amino acid sequence of SEQ ID NO: 4 (SEQ ID NO: 5) is shown as the middle row, and the amino acid sequence of bovine angiogenin-1 precursor comprising the amino acid sequence of SEQ ID NO: 4 and additional 23 amino acid residues existing before the amino acid sequence of SEQ ID NO: 4, which are different from those of SEQ ID NO: 5 (SEQ ID NO: 6) is shown as the lower row. The underlined parts are portions that agreed with the sequence of the bovine angiogenin-1 in database search performed by using the results of mass spectrometry.

Fig. 7 shows the results of identification of angiogenin-2 obtained by mass spectrometry in which measurement was performed for the same spots obtained in the two-dimensional electrophoresis. The amino acid sequence of bovine angiogenin-2 (SEQ ID NO: 7) is shown as the upper row, and the amino acid sequence of bovine angiogenin-2 comprising the amino acid sequence of SEQ ID NO: 7 and additional 24 amino acid residues existing before the amino acid sequence of SEQ ID NO: 7 (SEQ ID NO: 8) is shown as the lower row. The underlined parts are portions that agreed with the sequence of the bovine angiogenin-2 in database search performed by using the results of mass spectrometry.

Modes for Carrying out the Invention

Hereafter, preferred embodiments of the present invention will be explained in detail. However, the present invention is not limited to the following preferred embodiments, and freely changed within such a range that the present invention is feasible. The percentages used for indicating quantitative ratios in this specification are used on the mass basis, unless especially indicated.

[Antimicrobial activity enhancing agent]

The antimicrobial activity enhancing agent of the present invention has an effect of enhancing the inherent antimicrobial activity of lactoferrin or a lactoferrin hydrolysate.

Although the antimicrobial activity enhancing agent of the present invention may consist only of the milk protein described below, it may contain other components, for example, components used for formulating a drug mentioned later, so long as it contains the milk protein as an active ingredient. Further, the antimicrobial activity enhancing agent of the present invention may contain a milk protein other than angiogenin-1 and/or angiogenin-2 (except for lactoferrin), so long as it contains angiogenin-1 and/or angiogenin-2 as an active ingredient. Purity of angiogenin-1 and/or angiogenin-2 based on the proteins contained in the antimicrobial activity enhancing agent is preferably 0.002% or more, more preferably 0.01% or more, still more preferably 0.1% or more, particularly preferably 1.0% or more. The amount of lactoferrin will be explained later.

The antimicrobial activity enhancing agent of the present invention may be mixed with lactoferrin or a lactoferrin hydrolysate as liquids, or they may be mixed as powders. If they are made into powders and then mixed, operation for formulating a preparation such as tablet and troche becomes easier.

The antimicrobial activity enhancing agent of the present invention is characterized in that the milk protein as the active ingredient does not have antimicrobial activity, or even if it has an antimicrobial activity, it has an antimicrobial activity lower than that of lactoferrin or a lactoferrin hydrolysate, or shows a limited antimicrobial spectrum. And the antimicrobial activity enhancing agent of the present invention has an action of enhancing the antimicrobial activity of lactoferrin or a lactoferrin hydrolysate, when it is used together with lactoferrin or the lactoferrin hydrolysate, or it is mixed with lactoferrin or the lactoferrin hydrolysate, although it per se does not have antimicrobial activity. Further, even if the antimicrobial activity enhancing agent of the present invention per se has an antimicrobial activity, it provides an antimicrobial activity higher than the sum of the antimicrobial activities of the antimicrobial activity enhancing agent and lactoferrin or a lactoferrin hydrolysate, when it is used together with lactoferrin or the lactoferrin hydrolysate, or it is mixed with lactoferrin or the lactoferrin hydrolysate. The expression "enhancement of antimicrobial activity" includes both of the aforementioned cases.

As the effect of lactoferrin or a lactoferrin hydrolysate containing the antimicrobial activity enhancing agent according to an embodiment of the present invention, it can reduce number of *Escherichia coli* to at least 1/7 or less of that obtainable with lactoferrin or the lactoferrin hydrolysate not containing the antimicrobial activity enhancing agent, as demonstrated in the test examples mentioned later. In other words, it can reduce amount of *Escherichia coli* represented by CFU (colony forming unit) to 1/7 or less.

Further, as the effect of lactoferrin or a lactoferrin hydrolysate containing the antimicrobial activity enhancing agent according to another embodiment of the present invention, it can reduce number of *Escherichia coli* to at least 1/35 or less of that obtainable with lactoferrin or a lactoferrin hydrolysate not containing the antimicrobial activity enhancing agent.

That is, as for enhancement of the antimicrobial activity by the antimicrobial activity enhancing agent of the present invention, the antimicrobial activity enhancing agent of the present invention has a property that it can enhance the antimicrobial activity of lactoferrin or a lactoferrin hydrolysate of a specific amount against *Escherichia coli* preferably 7 times or more, more preferably 35 times or more, when the antimicrobial activity enhancing agent and lactoferrin or a lactoferrin hydrolysate are used together.

When the antimicrobial activity enhancing agent does not have antimicrobial activity against a certain microorganism, if number of a microorganism is decreased to 1/n by a specific amount of lactoferrin or a lactoferrin hydrolysate, and number of the microorganism is decreased to 1/m (provided that m > n) by lactoferrin or the lactoferrin hydrolysate and the antimicrobial activity enhancing agent used together, it means that the antimicrobial activity of lactoferrin or the lactoferrin hydrolysate is enhanced m/n times. Even when the antimicrobial activity enhancing agent has an antimicrobial activity against the microorganism, if it is sufficiently lower than the antimicrobial activity of lactoferrin or the lactoferrin hydrolysate, degree of the enhancement is approximated by m/n.

Degree of the enhancement of the antimicrobial activity is preferably evaluated under a condition providing the highest antimicrobial activity determined by adding the antimicrobial activity enhancing agent of the present invention in various amounts to a constant amount of lactoferrin or a lactoferrin hydrolysate. However, when the antimicrobial activity enhancing agent has an antimicrobial activity, it is a premise that the antimicrobial activity obtained when the antimicrobial activity enhancing agent and lactoferrin or a lactoferrin hydrolysate are used together is higher than the sum of the antimicrobial activities of the same amounts of antimicrobial activity enhancing agent and lactoferrin or the lactoferrin hydrolysate.

The antimicrobial activity enhancing agent of the present invention can be used also as a pharmaceutical composition for enhancing antimicrobial action, an antimicrobial action enhancing agent and an additive for enhancing antimicrobial action.

In the present invention, the term "antimicrobial activity" means a concept including activities for suppressing proliferation of a microorganism (antimicrobial activity), eliminating microorganisms from an object to reduce the number thereof (decolonization activity) and killing microorganisms (sterilization activity).

Therefore, the antimicrobial activity enhancing agent of the present invention may also be referred to as decolonization adjuvant or sterilization adjuvant. Similarly, the antimicrobial activity enhancing agent of the present invention may also be referred to as decolonization composition or sterilization composition.

In the present invention, the microorganism as the object of the antimicrobial activity to be enhanced is not particularly limited, so long as it is a microorganism against which lactoferrin or a lactoferrin hydrolysate shows antimicrobial activity. Examples include Gram-negative bacteria such as *Escherichia, Salmonella, Listeria, Bacillus, Cronobacter, Klebsiella* and *Pseudomonas* bacteria, Gram-positive bacteria such as *Staphylococcus* and *Streptococcus* bacteria, fungi such as *Candida* yeasts, and so forth.

[Milk protein]

The milk protein as the active ingredient of the present invention can be prepared by using colostrum, transitional milk, normal milk, late lactation milk or the like of a mammal (for example, human, cow, water buffalo, horse, goat, sheep etc.), or skim milk which is a processed product of the foregoing milks, as raw milk, and subjecting it to a cation exchange treatment or a ultrafiltration membrane treatment.

Further, the milk protein can be prepared from any raw milk containing whey (milk serum) proteins without particular limitation.

Furthermore, the milk protein is not limited to a protein obtained from milk or a dairy product as a raw material, but may be a protein obtained from a body fluid other than milk or a tissue of animals, a chemically synthesized product or a recombinant protein, so long as it has a structure equivalent to a protein contained in milk. Further, angiogenin-1 and angiogenin-2, or fragments thereof are not limited to those having any of the amino acid sequences of SEQ ID NOS: 4 to 8, but may be a conservative variant thereof. Examples of the conservative variant thereof include a protein having any of the amino acid sequences of SEQ ID NOS: 4 to 8 including substitution, deletion, insertion, addition, inversion or the like of one or several amino acid residues, for example, 1 to 10 amino acid residues, preferably 1 to 5 amino acid residues, more preferably 1 to 3 amino acid residues, in the sequence, and a protein having a homology of 80% or more, preferably 90% or more, more preferably 95% or more, to any one of the amino acid sequences of SEQ ID NOS: 4 to 8, and having an action of enhancing the antimicrobial activity of lactoferrin or a lactoferrin hydrolysate.

[Lactoferrin]

As lactoferrin used in the present invention as the object of enhancement of antimicrobial activity, any of marketed lactoferrin, lactoferrin isolated from colostrum, transitional milk, normal milk, late lactation milk or the like of a mammal (for example, human, cow, water buffalo, horse, goat, sheep etc.), skim milk, whey etc. which are processed products of the foregoing milks and so forth by a conventional method such as ion exchange chromatography, apolactoferrin produced by deironization of lactoferrin in a conventional manner, and metal-unsaturated or saturated lactoferrin produced by partially or fully chelating apolactoferrin with a metal such as iron, copper, zinc and manganese may be used.

Further, human lactoferrin produced by a recombinant fungus, a recombinant cow (transgenic cow) or the like obtained by a recombinant DNA technique, and so forth can be similarly used for the present invention.

[Lactoferrin hydrolysate]

In the present invention, as the lactoferrin hydrolysate as the object of the enhancement of antimicrobial activity, a marketed product, or a hydrolysate obtained by hydrolyzing the lactoferrin defined in the present invention with an acid or an enzyme can be used, and it can be obtained by, for example, the method described in Japanese Patent Application No. 3-171736.

In addition, in the present invention, the lactoferrin hydrolysate as the object of the enhancement of antimicrobial activity also includes a lactoferrin peptide isolated or purified from a lactoferrin hydrolysate.

Examples of the lactoferrin peptide include, for example, Lactoferricin (registered trademark).

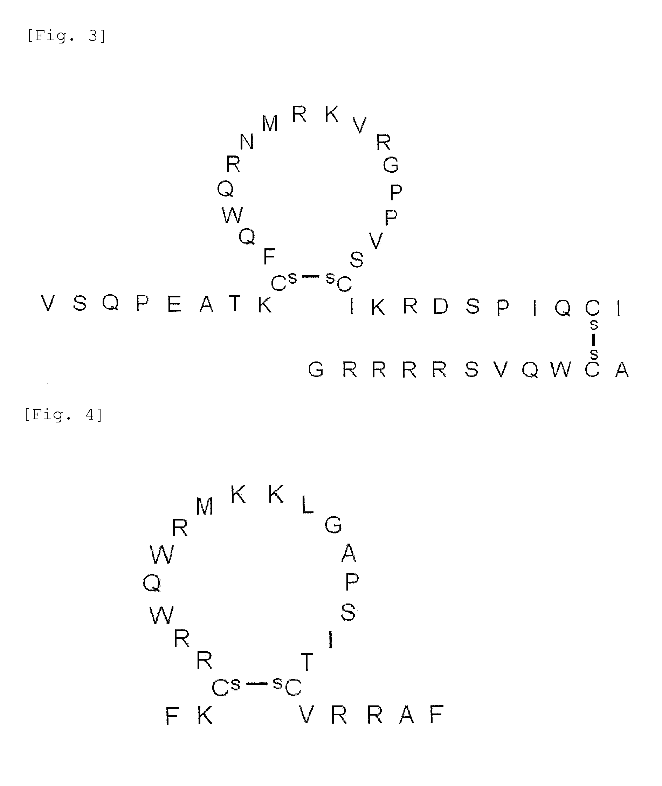

Fig. 3 shows the primary structure of human Lactoferricin (Lactoferricin H) (amino acid sequence: SEQ ID NOS: 1 and 2), and Fig. 4 shows the primary structure of bovine Lactoferricin (Lactoferricin B) (amino acid sequence: SEQ ID NO: 3).

When lactoferrin is hydrolyzed with an acid, lactoferrin is made into an aqueous solution, an inorganic acid or an organic acid is added to the solution, and the mixture is heated at a predetermined temperature for a predetermined time to hydrolyze lactoferrin. When the hydrolysis is performed with an enzyme, lactoferrin is made into an aqueous solution, the solution is adjusted to the optimum pH of an enzyme to be used, an enzyme such as pepsin and trypsin is added to the solution, and the mixture is maintained at a predetermined temperature for a predetermined time to perform the hydrolysis. After the hydrolysis, the enzyme is inactivated in a conventional manner. The hydrolysate obtained by the hydrolysis with an acid or enzyme is a mixture of hydrolysates of various molecular weights containing a peptide having antimicrobial activity. Degree of the decomposition by the aforementioned hydrolysis is desirably in the range of 6 to 20%. This degree of decomposition is calculated in accordance with the following equation from total nitrogen amount in a sample measured by the Kjeldahl method, and formol nitrogen amount in the sample measured by the formol titration method.
```
Degree of decomposition = (Formol nitrogen amount/Total
nitrogen amount) x 100
```

The reaction mixture obtained by the aforementioned hydrolysis with an acid or enzyme (solution of lactoferrin hydrolysates) is cooled in a conventional manner, neutralized, desalted and decolorized in a conventional manner as required, and further fractionated as required, and the obtained solution as it is, or as a concentrate obtained by concentrating the solution, or powder obtained by concentrating the solution and lyophilizing the residue is used. Further, as described above, an antimicrobial peptide such as Lactoferricin may be isolated from the hydrolysate.

[Active ingredient of the present invention]

The active ingredient of the antimicrobial activity enhancing agent of the present invention consists of one or more kinds of milk protein components, and these one or more components have an action and effect of enhancing the antimicrobial activity of lactoferrin or a lactoferrin hydrolysate.
The "milk protein" as the active ingredient may consist of a single kind of protein or a mixture of two or more kinds of proteins, so long as it has an action of enhancing the antimicrobial activity of lactoferrin or a lactoferrin hydrolysate.
The inventors of the present invention elucidated that the milk protein as the component that enhances antimicrobial activity has, at least, 1) an action of enhancing the antimicrobial activity of lactoferrin or a lactoferrin hydrolysate, 2) a molecular weight of 30 kDa or less, and 3) an isoelectric point of 9.0 to 11.0.
Further, the milk protein may contain lactoferrin or a lactoferrin hydrolysate at a content of 2.0 mass % or less based on the total amount of milk proteins. Although it is preferred that the milk protein that can serve as the active ingredient of the present invention does not contain lactoferrin or a lactoferrin hydrolysate at all, it has been confirmed that, if ratio of lactoferrin or a lactoferrin hydrolysate in the milk protein as the active ingredient is 2.0% or less in terms of mass ratio, the milk protein as a whole does not show antimicrobial activity.
Furthermore, it was estimated in Example 1 described later that the antimicrobial activity was enhanced by one kind of component or a combination of two or more kinds of components contained in the spots obtained in two-dimensional electrophoresis. Further, it was also confirmed that angiogenin-1 or a mixture of angiogenin-1 and angiogenin-2 enhanced the antimicrobial activity of lactoferrin or a lactoferrin hydrolysate.
Specific examples of the milk protein as the active ingredient of the antimicrobial activity enhancing agent of the present invention include angiogenin-1, angiogenin-2, and a mixture of these. The isoelectric points of bovine angiogenin-1 and angiogenin-2 expected from the amino acid sequences are 9.1 and 9.8 to 10, respectively. The bovine angiogenin-1 and angiogenin-2 do not substantially have antimicrobial activity against *Escherichia coli.*
The milk protein may be a dimer of angiogenin-1 and/or angiogenin-2, a fragment thereof, or a mixture of these, so long as it has an action of enhancing the antimicrobial activity of lactoferrin or a lactoferrin hydrolysate.

[Amount of antimicrobial activity enhancing agent]

When the antimicrobial activity enhancing agent of the present invention is used for lactoferrin or a lactoferrin hydrolysate, mass ratio of the milk protein in the antimicrobial activity enhancing agent and lactoferrin or a lactoferrin hydrolysate is preferably 1:80 to 1:1, more preferably 1:80 to 1:10, still more preferably 1:60 to 1:10, further preferably 1:40 to 1:10.
Further, when the antimicrobial activity enhancing agent of the present invention is used together with, or mixed with a lactoferrin hydrolysate, in particular with Lactoferricin (registered trademark), which is a peptide derived from lactoferrin, they are preferably used at a ratio of the milk protein in the antimicrobial activity enhancing agent and Lactoferricin of 1:1 to 4:1, more preferably at a ratio of the milk protein in the antimicrobial activity enhancing agent and Lactoferricin of 1.5:1 to 2.5:1.

[Antimicrobial composition]

The antimicrobial composition of the present invention contains the antimicrobial activity enhancing agent of the present invention, and one or plural kinds of substances selected from the group consisting of lactoferrin and a lactoferrin hydrolysate, and is characterized in that mass ratio of the milk protein in the antimicrobial activity enhancing agent to lactoferrin and/or the lactoferrin hydrolysate is 1/80 or more. Although the mass ratio of the milk protein in the antimicrobial activity enhancing agent to lactoferrin and the lactoferrin hydrolysate is not particularly limited so long as it is 1/80 or more, it is preferably 1/40 or more, more preferably 1/20 or more.
Although the maximum mass ratio of the milk protein in the antimicrobial activity enhancing agent to lactoferrin and the lactoferrin hydrolysate is not particularly limited, high antimicrobial activity can be expected even with a ratio of, for example, 1/1 or less, 1/2 or less, 1/5 or less, or 1/10 or less.
Mass ratio of angiogenin and lactoferrin contained in ordinary whey is about 1:200.
The antimicrobial composition of the present invention can be blended in drugs, foods or drinks, feeds, cosmetics, quasi drugs, and so forth.

[Drug]

The drug of the present invention is characterized by containing the antimicrobial composition of the present invention or the antimicrobial activity enhancing agent of the present invention, and lactoferrin or a lactoferrin hydrolysate. Form of the drug of the present invention is not particularly limited, and it can be formulated in various forms by known methods, and administered. In a preferred embodiment, it can be orally administered. When the drug is formulated, the antimicrobial activity enhancing agent of the present invention and lactoferrin or a lactoferrin hydrolysate are mixed and used.
For example, the drug can be formulated with the antimicrobial activity enhancing agent of the present invention and lactoferrin or a lactoferrin hydrolysate by using an arbitrary additive such as a pharmacologically acceptable excipient. When the drug is formulated, content of lactoferrin or a lactoferrin hydrolysate in the drug is not particularly limited. Although it is basically considered that the antimicrobial activity enhancing agent of the present invention and lactoferrin or a lactoferrin hydrolysate scarcely show adverse reaction, content of the composition comprising the antimicrobial activity enhancing agent and lactoferrin or a lactoferrin hydrolysate in the drug is usually 0.1 to 60 mass %, preferably 10 to 50 mass %. Further, content of the composition comprising the antimicrobial activity enhancing agent and Lactoferricin (registered trademark) in the drug is usually 0.01 to 6 mass %, preferably 1 to 5 mass %.
The ratio of amounts of the antimicrobial activity enhancing agent and lactoferrin and a lactoferrin hydrolysate is the same as that described for the antimicrobial composition.
When the drug is formulated, additives such as excipient, binder, disintegrant, lubricant, stabilizer, corrigent, diluent and solvent for injections can be used. Specific examples of the formulation include tablets (including sugar-coated tablets, enteric coated tablets and buccal tablets), powders, capsules (including enteric capsules and soft capsules), granules (including coated granules), pills, troches, enclosed liposome agents, solutions, pharmaceutically acceptable sustained release preparations of these, and so forth.
Examples of the carrier and excipient used for these formulations include lactose, glucose, sucrose, mannitol, potato starch, corn starch, calcium carbonate, calcium phosphate, calcium sulfate, crystalline cellulose, glycyrrhizae radix pulverata, gentianae radix pulverata, and so forth, and examples of the binder include starch, gelatin, syrup, polyvinyl alcohol, polyvinyl ether, polyvinylpyrrolidone, hydroxypropylcellulose, ethylcellulose, methylcellulose, carboxymethylcellulose, and so forth.
Examples of the disintegrant include starch, agar, gelatin powder, carboxymethylcellulose sodium, carboxymethylcellulose calcium, crystalline cellulose, calcium carbonate, sodium hydrogencarbonate, sodium arginate, and so forth.
Examples of the lubricant include magnesium stearate, hydrogenated vegetable oil, Macrogol, and so forth, and examples of the colorant include Red No. 2, Yellow No. 4, Blue No. 1, and so forth, which are allowed to be added to drugs.
Tablets and granules can be coated with sucrose, hydroxypropylcellulose, purified shellac, gelatin, sorbitol, glycerol, ethylcellulose, hydroxypropylcellulose, hydroxypropylmethylcellulose, polyvinylpyrrolidone, cellulose acetate phthalate, hydroxypropylmethylcellulose phthalate, methyl methacrylate, methacrylic acid polymer, and so forth, as required.

Further, the pharmaceutical composition used together may be contained in the drug of the present invention as an active ingredient, or may not be contained in the drug of the present invention, but may be prepared as a separate drug and combined with the drug to provide a commercial product.

Examples of the pharmaceutical composition for antimicrobial use include drugs and quasi drugs such as dentifrice, and mouthwash.

[Food or drink]

The food or drink of the present invention is characterized by containing the antimicrobial composition of the present invention or the antimicrobial activity enhancing agent of the present invention, and lactoferrin or a lactoferrin hydrolysate.

Content of the composition comprising the antimicrobial activity enhancing agent and lactoferrin or a lactoferrin hydrolysate in the food or drink of the present invention is usually 0.1 to 60 mass %, preferably 10 to 50 mass %. Content of the composition comprising the antimicrobial activity enhancing agent and Lactoferricin (registered trademark) in the food or drink is usually 0.01 to 6 mass %, preferably 1 to 5 mass %.

Ratio of amounts of the antimicrobial activity enhancing agent and lactoferrin and a lactoferrin hydrolysate is the same as that explained for the antimicrobial composition.

Form of the food or drink of the present invention is not particularly limited, and examples include soft drinks, milk beverages etc. or concentrated undiluted solutions or powders for preparation of these drinks; dairy products such as processed milk and fermented milk; infant formula; enteral formula; functional foods; and so forth, as well as drinks such as carbonated drinks, nutritious drinks and fruit drinks (including concentrated undiluted solutions or powders for preparation of these drinks); frozen desserts such as ice cream, ice sherbet and chipped ice; noodles such as buckwheat noodles, udon, bean thread noodles, wrapping sheets for Chinese meat dumpling, wrapping sheets for steamed meat dumpling, Chinese noodles and instant noodles; confectioneries such as candy, chewing gum, candy, chocolate, tablet confectioneries, snacks, biscuits, jelly, jam, cream and baked confectioneries; aquatic and livestock processed foods such as boiled fish paste, ham and sausage; fats, oils, and fat or oil processed foods such as salad oil, tempura oil, margarine, mayonnaise, shortening, whipped cream and dressing; seasonings such as sauce and dipping sauce; soup, stew, salad, daily dish, pickles, bread, and so forth, in which powder of lactoferrin or a lactoferrin hydrolysate or an aqueous solution thereof (syrup etc.) is formulated together with the antimicrobial activity enhancing agent of the present invention.

Such foods and drinks can be produced by, for example, blending lactoferrin (including lactoferrin in the form of lactoferrin powder, lactoferrin aqueous solution (syrup etc.), and so forth) with the antimicrobial activity enhancing agent of the present invention, as well as saccharides such as dextrin and starch; proteins such as gelatin, soybean protein and corn protein; amino acids such as alanine, glutamine and isoleucine; polysaccharides such as cellulose and gum arabic; oil or fat such as soybean oil and medium chain fatty acid triglyceride, and so forth.

Further, preferred examples of the form of the food or drink include supplement in the form of a liquid or tablet, and so forth.

[Feed]

The feed of the present invention is characterized by containing the antimicrobial composition of the present invention or the antimicrobial activity enhancing agent of the present invention and lactoferrin or a lactoferrin hydrolysate.

Content of the composition comprising the antimicrobial activity enhancing agent and lactoferrin or a lactoferrin hydrolysate in the feed of the present invention is usually 0.1 to 60 mass %, preferably 10 to 50 mass %. Content of the composition comprising the antimicrobial activity enhancing agent and Lactoferricin (registered trademark) in the feed is usually 0.01 to 6 mass %, preferably 1 to 5 mass %.

Ratio of amounts of the antimicrobial activity enhancing agent and lactoferrin or a lactoferrin hydrolysate is the same as that explained for the antimicrobial composition.

Form of the feed of the present invention is not particularly limited. For example, powder of lactoferrin or a lactoferrin hydrolysate or an aqueous solution thereof (syrup etc.) can be blended together with the antimicrobial activity enhancing agent of the present invention, and the feed can be produced by, for example, blending the antimicrobial activity enhancing agent of the present invention and lactoferrin with cereals such as corn, wheat, barley, rye and milo; vegetable oil meals such as soybean oil meal, rapeseed oil meal, coconut oil meal and linseed oil meal; brans such as bran, wheat bran, rice bran and defatted rice bran; production meals such as cone gluten meal and corn jam meal; animal-derived feeds such as fish meal, skim milk powder, whey, yellow grease and tallow; yeasts such as torula yeast and brewer's yeast; mineral material feeds such as calcium triphosphate and calcium carbonate; oils and fats; simple substance amino acids; saccharides, and so forth. Examples of the form of the feed include pet food, livestock feed, fish breeding feed, and so forth.

[Method for producing antimicrobial activity enhancing agent]

The antimicrobial activity enhancing agent of the present invention can be produced by the method comprising the following steps.

That is, it is a method for producing an antimicrobial activity enhancing agent for lactoferrin or a lactoferrin hydrolysate comprising the aforementioned milk protein as an active ingredient, which comprises: (a) the step of contacting raw milk with a cation exchange resin to adsorb proteins in the raw milk to the cation exchange resin, (b) the step of washing the cation exchange resin having undergone the aforementioned adsorption treatment, and flowing an elution solvent to elute a protein fraction having an isoelectric point of 9.0 to 11.0, and (c) the step of subjecting the eluted protein fraction to membrane filtration using an ultrafiltration membrane having a molecular weight cutoff of 30 kDa to prepare a milk protein having the following properties:

> (A) an action of enhancing the antimicrobial activity of lactoferrin or a lactoferrin hydrolysate,
> (B) a molecular weight of 30 kDa or less,
> (C) an isoelectric point of 9.0 to 11.0.

The production method of the present invention may further comprise, following the aforementioned step of (c), (d) the step of flowing the passed-through solution through an ultrafiltration membrane having a molecular weight cutoff of 10 kDa to collect a concentrate of a fraction of 10 kDa or more.

The production method of the antimicrobial activity enhancing agent of the present invention partially overlaps with the conventional methods for producing lactoferrin or lactoperoxidase, but it is different in that it comprises the steps of (b) to (d).

In the production method of the present invention, only low molecular weight proteins of 30 kDa or less are collected, and therefore lactoferrin or lactoperoxidase, each of which has a molecular weight of about 80,000 daltons, is scarcely contained in the antimicrobial activity enhancing agent of the present invention.

That is, the antimicrobial activity enhancing agent of the present invention differs from lactoferrin or lactoperoxidase in respect of molecular weight.

The method for producing the antimicrobial activity enhancing agent of the present invention will be further explained in detail below.

First, raw milk is contacted with a cation exchanger to perform an adsorption treatment.

The raw milk is not particularly limited so long as a material containing a protein having the antimicrobial activity enhancing action is selected, and examples include raw milk of a mammal (for example, human, cow, water buffalo, horse, goat, sheep etc.), processed milk such as skim milk, fractionation products such as whey, milk produced by a recombinant cow (transgenic cow) or the like, and so forth. The milk may be any of colostrum, transitional milk, normal milk, late lactation milk, and so forth.

As the cation exchanger, one having a weakly acidic group or a strongly acidic group as the ion exchange group is used. As the weakly acidic ion exchange group or the strongly acidic ion exchange group, an ion exchange group having an objective function as an ion exchange group can be arbitrarily selected. Examples of the weakly acidic ion exchange group include carboxyl group, carboxymethyl group, and so forth. Examples of the strongly acidic ion exchange group include sulfopropyl group, and so forth.

The cation exchanger is not particularly limited, and can be arbitrarily selected as required. Preferred examples of the cation exchanger include those comprising porous particles consisting of a cross-linked polysaccharide (agarose, dextran, cellulose etc.), hydrophilic silica gel, synthetic polymer, or the like, introduced with weakly acidic or strongly acidic ion exchange groups. Specific examples of the weakly acidic ion exchanger include Sepabeads FP-CM13 (Mitsubishi Chemical, exchange group: carboxymethyl group), CM-Sephadex C-50 (GE Healthcare, exchange group: carboxymethyl group) and CM-Sepharose FF (GE Healthcare, exchange group: carboxymethyl group). Specific examples of the strongly acidic ion exchanger include SP-Sepharose FF (GE Healthcare) and SP-Sepharose Big Beads (GE Healthcare).

Form, size, surface condition, material etc. of the resin of the cation exchanger are not limited, and may be arbitrarily selected as required. Examples of the form of the cation exchanger for use include a pre-packed column already filled with an ion exchanger resin, a medium such as column filled with resin beads of a cation exchanger, and so forth. When these forms are used, it is preferred that one kind of cation exchanger is used in combination with one kind of medium in view of simplicity. However, two or more media each filled with resin beads may be connected in series or in parallel to perform chromatography, if needed. Although the form of the medium may be arbitrarily selected as required, a form allowing easy washing thereof and providing a larger contact area for resin beads or the like to be contained is preferred, and internal surface thereof is preferably a flat surface having no unevenness.

Specifically, a shape having a circularly curved side such as cylindrical shape (cylindrical column, rod) and conical shape is preferably used as the shape of the medium. The material of the medium may be arbitrarily selected, and stainless steel, glass, polypropylene, polyethylene, polyethylene terephthalate, polycarbonate, acrylic resin and for forth can be preferably used. Size of the medium may be appropriately selected depending on the scale of the process, and it can be arbitrarily selected from the range of, for example, several cubic centimeters to several cubic meters. Examples of commercially available pre-packed column that can be preferably used for the present invention include Hiprep CMFF16/10 (GE Healthcare, exchange group: carboxymethyl group), Hiprep SPFF16/10 (GE Healthcare, exchange group: sulfopropyl group), TOYOPEARL CM-650 Series (TOSOH, exchange group: carboxymethyl group), TOYOPEARL SP-650 Series (TOSOH, exchange group: sulfopropyl group), and so forth.

The method of the adsorption treatment (contact) for the raw milk and the cation exchanger may be arbitrarily selected. The adsorption treatment can be performed by such a method as the batch type stirring method and continuous column method. Any method may be employed so long as the raw milk and the cation exchanger can fully be contacted. The adsorption treatment may consist of one adsorption treatment, or a combination of two or more adsorption treatments.

In the case of batch type process, it is appropriate to use a large amount of the cation exchanger when high yield is desired from a certain fixed amount of the raw milk, or to use a large amount of the raw milk when it is desired to obtain high yield by using a certain fixed amount of the cation exchanger. The mixing volume ratio of the raw milk and the cation exchanger in the case of batch type process can be arbitrarily adjusted depending on adsorption ability of the cation exchanger and type of specific method used for the adsorption treatment.

Cation exchangers are roughly classified into two types, those of hard type and soft type, according to the characteristics thereof. In the present invention, those of both types can be used.

Cation exchangers of the hard type hardly show change of volume of the cation exchangers themselves depending on ionic strength or pH, and also hardly show change of volume of the cation exchangers themselves even if pressure applied to the cation exchangers is changed with change of flow rate or the like. Therefore, cation exchangers of the hard type are more suitable for the continuous column method in which a cation exchanger is retained in a column, and a solution is flown through it at a high flow rate.

On the other hand, cation exchangers of the soft type show significant change of volume of the cation exchangers themselves depending on ionic strength or pH, and also easily show change of volume of the cation exchangers themselves when pressure applied to the cation exchangers is changed with change of flow rate or the like. Therefore, it is difficult to retain a cation exchanger of the soft type in a column, and flow a solution through it at a high flow rate. In particular, when skim milk, whey or the like is flown through the cation exchanger, a larger pressure loss occurs in a cation exchanger layer compared with the case of flowing other salt solutions through it. Therefore, cation exchangers of the soft type are generally suitable for the batch type process.

Among those mentioned above, Sepabeads FP-CM13 (Mitsubishi Chemical), CM-Sepharose FF (GE Healthcare), SP-Sepharose FF (GE Healthcare) and SP-Sepharose Big Beads (GE Healthcare) are cation exchangers of the hard type, and CM-Sephadex C-50 (Amersham) is a cation exchanger of the soft type.

If the adsorption treatment of the raw milk and the cation exchanger is performed at a temperature lower than 0°C, the raw milk may be frozen, and viscosity thereof may be increased, and if it is performed at a temperature exceeding 60°C, proteins in the milk, for example, basic proteins in the milk, may be denatured. Therefore, the treatment is preferably performed at a temperature in the range of 0 to 60°C. In addition, even within such a range, when the treatment is performed at a temperature of 25 to 60°C, if the adsorption treatment takes a long time, proteins in the milk, for example, basic proteins in the milk, may be gradually denatured. Therefore, the treatment is especially preferably performed at a temperature of 0 to 25°C. Further, when unsterilized raw milk is used, the treatment is preferably performed at a temperature of 0 to 10°C in order to prevent proliferation of bacteria.

Time of the adsorption treatment (contact time) of the raw milk and the cation exchanger may be appropriately selected, in consideration of temperature for the adsorption treatment, method of the adsorption treatment (batch type process or continuous column method) to be employed, etc. For example, in the case of the batch type process, time of the adsorption treatment of the raw milk and the cation exchanger is preferably 1 minute or more to 24 hours or less, more preferably 10 minutes or more to 6 hours or less. Further, in the case of the continuous column method, linear flow rate is preferably 10 cm/h or more to 1000 cm/h or less.

Then, the cation exchanger having undergone the adsorption treatment is subjected to a washing treatment. As a washing solution for this treatment, a salt solution having a low ionic strength lower than 0.07 or a neutral or weakly acidic buffer may be used. However, in view of the production cost, washing is preferably performed with water.

The raw milk used may be removed from the cation exchanger having undergone the adsorption treatment by any washing method. For example, only the cation exchanger is transferred from a container containing the raw milk and the cation exchanger, and washed in another place, or the raw milk may be removed from such a container as mentioned above, and then the cation exchanger may be washed in the container.

Then, an elution solvent is contacted with the cation exchanger having undergone the washing treatment. The target protein is thereby eluted from the cation exchanger into the elution solvent to obtain eluate.

The elution solvent used in this step preferably has an ionic strength of 0.07 or more to 1.7 or less, more preferably of 0.16 or more to 1.0 or less, particularly preferably of 0.25 or more to 0.6 or less. An elution fraction having an isoelectric point of 9.0 to 11.0 can be thereby obtained.

As the elution solvent, a neutral or weakly acidic buffer of which ionic strength is adjusted to be within the aforementioned range can also be used. However, it is more preferable to use an aqueous solution dissolving only salts (salt solution), if the production cost is taken into consideration. As the salt used for the present invention, one or plural kinds of salts may be arbitrarily selected as required.

Preferred salt solution is a solution of a salt containing one kind of salt or a mixture of two or more kinds of salts selected from the group consisting of sodium chloride, potassium chloride, calcium chloride, magnesium chloride, and so forth, and specifically, 1 to 5% solution of sodium chloride, preferably 1.5 to 3.5% solution of sodium chloride, and so forth can be exemplified.

Then, the obtained eluate is subjected to a membrane treatment performed by a membrane separation method using an ultrafiltration membrane. The eluate is thereby concentrated to produce precipitates in the eluate.

Operation methods for ultrafiltration membranes are classified into two types, i.e., the usual ultrafiltration in which water and components having a molecular weight below the molecular weight cutoff are passed through the membrane to attain the removal, and the running water filtration method (diafiltration) in which the operation is continuously performed with adding water in a volume equal to the solution passed through the membrane to the solution retained on the membrane. For the present invention, both of the methods can be used. In particular, the latter running water filtration method is more preferred, because a desalting treatment can be performed simultaneously with concentration, and low molecular weight components can be fully removed from the retained solution.

When the former usual ultrafiltration method is used, it is preferable to perform a desalting treatment by such a method as dialysis or gel filtration, after the ultrafiltration.

As the ultrafiltration membrane, any of commercially available ultrafiltration membranes can be used. Specific examples include IRIS3038 and IRIS3072 membranes (both from Rhone-Poulenc S.A.), GR-61pp membrane (DDS), and so forth, and all of these have a property of a fractionation molecular weight of 30 kDa (molecular weight cutoff of 30 kDa).

As the material of the ultrafiltration membrane, any of organic materials and inorganic materials may be used, and the material can be selected in consideration of cost, availability etc.

The ultrafiltration treatment can be performed at any temperature of elution solution so long as it is lower than heat resistant temperature of the membrane used (for example, 80°C or less in the case of GR-61pp membrane). However, in order to prevent denaturation of proteins, it is preferable to perform at 0 to 60°C, for example, 25 to 60°C, or 0 to 25°C.

The ultrafiltration may be performed at any pressure lower than pressure resistance limit of the membrane used (for example, 0.6 MPa or less in the case of the GR-61pp membrane). However, use of the membrane at a pressure near the pressure resistance limit may shorten the life of the membrane, and therefore the treatment is preferably performed at a pressure of 2/3 or less of the pressure resistance limit (for example, 04 MPa in the case of the GR-61pp membrane).

Any of forms of ultrafiltration membrane modules, for example, those of tube type, hollow fiber type, flat membrane type, spiral type, etc., may be used. When an internal pressure type hollow fiber module is used, generation of precipitates in hollow fibers may cause obstruction of flow paths, and therefore the treatment is preferably performed in consideration of the pressure etc.

Examples

Hereafter, the present invention will be explained in detail with reference to examples and test examples. However, the present invention is not limited to the following examples.

First, measurement methods and test methods used in the examples and the test examples will be explained.

[Two-dimensional electrophoresis]

In order to determine component composition of the antimicrobial activity enhancing agent of the present invention, two-dimensional electrophoresis was performed by using ZOOM IPG Runner System (Invitrogen). For the first dimensional electrophoresis, ZOOM Strip pH3-10NL for the pH range of 3 to 10 was used. For the second dimensional electrophoresis, NuPAGE 4-12% Bis-Tris ZOOM Gel was used. Staining was performed by using SimplyBlue SafeStain (Invitrogen).

[Mass spectrometry]

The stained gel obtained from the two-dimensional electrophoresis was imaged with Densitograph (ATTO), and relative quantification was performed by using analysis software. The obtained spots were excised from the gel, the objective protein was decolorized, reductively alkylated, digested in the gel by trypsinization, and subjected to mass spectrometry using a MALDI-TOF/MS system (trade name: AutoflexII, BRUKER), and the protein was identified by searching the database of NCBI (National Center for Biotechnology Information, http://www.ncbi.nlm.nih.gov/) using the MASCOT software produced by Matrix Science.

[Antimicrobial activity enhancement test]

In the antimicrobial activity enhancement test, antimicrobial activity of a sample obtained by mixing the antimicrobial activity enhancing agent of the present invention with lactoferrin or a lactoferrin hydrolysate was measured. As a control, antimicrobial activity of a sample containing only lactoferrin or a lactoferrin hydrolysate was also measured. Specific procedure of the test was as follows.

(1) Preparation of sample

At least the following samples were prepared. For the test of lactoferrin, two kinds of Samples 2 having different concentrations of the antimicrobial activity enhancing agent were prepared.

    Sample 1: Lactoferrin, lactoferrin hydrolysate, or the like
    Sample 2: Mixture of lactoferrin, lactoferrin hydrolysate, or the like and antimicrobial activity enhancing agent
    Sample 3: Antimicrobial activity enhancing agent

(2) Test method

By using 1% Bacto peptone medium, *Escherichia coli* K-12 strain was inoculated into the medium contained in a polystyrene tube with a 5 ml capacity, and precultured in an incubator at 37°C for 6 hours to obtain a cell suspension for the test. The precultured *Escherichia coli* was added to a 96-well plate at a cell density of $10^6$ cells/mL together with each of the aforementioned samples, and cultured at 37°C for a predetermined time. After the culture for the predetermined time, the culture medium containing *Escherichia coli* was serially diluted with 0.85% physiological saline, and plated on an agar medium, and culture was performed at 37°C for a predetermined time. Then, number of colonies (colony forming units : CFU) was measured.
In addition, *Escherichia coli* was cultured only with the medium as a control, besides the culture using the aforementioned samples.

(3) Results

Antimicrobial activity of each sample was evaluated on the basis of the value of CFU. Degree of enhancement of the antimicrobial activity was calculated by comparison of the numbers of *Escherichia coli* (CFU) that remained after the test. For example, if CFU of Sample 1 is 100, and CFU of Sample 2 is 10 after the test, the antimicrobial activity of Sample 2 can be determined to be 10 times that of Sample 1.

[Example 1]

In this example, the antimicrobial activity enhancing agent of the present invention was produced.

(1) Test method

As the cation exchange resin, CM-Sephadex C-50 (GE Healthcare) was used. 20 L of unsterilized skim milk was flown through a column filled with 170 mL of the cation exchange resin to adsorb milk proteins on the exchange resin. Then, the cation exchange resin in the column was sufficiently washed with deionized water, and 0.27 M sodium chloride solution (Kokusan Chemical) was flown through the column to elute the proteins adsorbed on the resin. Subsequently, 1.7 M sodium chloride solution was flown as an elution solvent to elute a fraction of the proteins adsorbed on the resin. This eluate was desalted by an ultrafiltration treatment using a membrane having a molecular weight cutoff of 10 kDa, concentrated and lyophilized in a conventional manner to obtain powder of the protein fraction.
160 mg of the lyophilized protein powder was dissolved in 10 mL of 0.5 M sodium chloride solution, the solution was centrifuged (4,000 x g, 15 minutes) using a centrifugal ultrafiltration membrane having a molecular weight cutoff of 30 kDa (trade name: Amicon Ultra, Millipore), and a basic protein fraction of the permeate was collected. Further, 10 mL of 0.5 M sodium chloride solution was added to the centrifugal ultrafiltration membrane and centrifuged (4,000 x g, 15 minutes), and a basic protein fraction of the permeate was collected. This basic protein fraction and the previously obtained basic protein fraction were mixed, desalted and concentrated with a centrifugal ultrafiltration membrane having a molecular weight cutoff of 10 kDa (trade name: Amicon Ultra, Millipore) to obtain an antimicrobial activity enhancing agent of the present invention as the concentrated fraction.

The molecular weight distribution of the antimicrobial activity enhancing agent of the present invention was 8 to 30 kDa as determined by two-dimensional electrophoresis, and isoelectric point of the same was 9.0 to 11.0.

[Example 2]

To 95 g of bovine lactoferrin (Morinaga Milk Industry), 5 g of an antimicrobial activity enhancing agent produced in the same manner as that of Example 1 was added, and they were uniformly mixed to prepare a drug for antimicrobial use in which the antimicrobial activity of lactoferrin was enhanced.

[Example 3]

As a lactoferrin hydrolysate, a lactoferrin hydrolysate powder obtained by adjusting the bovine lactoferrin used in Example 2 to pH 5 or less, enzymatically digesting it by reaction with 3% pepsin, concentrating the digestion product with a reverse osmotic membrane, and lyophilizing the concentrate was used.

To 95 g of the aforementioned lactoferrin hydrolysate powder, 5 g of an antimicrobial activity enhancing agent produced in the same manner as that of Example 1 was added, and they were uniformly mixed to prepare a drug for antimicrobial use in which the antimicrobial activity of the lactoferrin hydrolysate was enhanced.

[Example 4]

To 95 g of bovine lactoferrin (Morinaga Milk Industry), 5 g of an antimicrobial activity enhancing agent produced in the same manner as that of Example 1 was added, they were uniformly mixed, and the mixture was added in an amount of 10 mass % to fermented milk to prepare a fermented milk food.

Hereafter, the action of the antimicrobial activity enhancing agent of the present invention will be explained in detail with reference to test examples.

[Test Example 1]

In this example, an antimicrobial activity enhancing agent of the present invention was produced, and components thereof were identified.

(1) Test method

In order to determine components of the antimicrobial activity enhancing agent obtained in Example 1, two-dimensional electrophoresis was performed by using ZOOM IPG Runner System (Invitrogen). For the first dimensional electrophoresis, ZOOM Strip pH3-10NL for the pH range of 3 to 10 was used. For the second dimensional electrophoresis, NuPAGE 4-12% Bis-Tris ZOOM Gel was used. Staining was performed by using SimplyBlue SafeStain (Invitrogen).

The molecular weight distribution of the antimicrobial activity enhancing agent of the present invention was 8 to 30 kDa, and isoelectric point of the same was 9.0 to 11.0.

Further, the stained gel obtained from the two-dimensional electrophoresis was imaged with Densitograph (ATTO), and relative quantification was performed by using analysis software. The obtained spots were excised from the gel, the objective protein was decolorized, reductively alkylated, digested in the gel by trypsinization, and subjected to mass spectrometry using a MALDI-TOF/MS system (trade name: AutoflexII, BRUKER), and the protein was identified by searching the database of NCBI (National Center for Biotechnology Information, http://www.ncbi.nlm.nih.gov/) using the MASCOT software produced by Matrix Science.

(2) Results

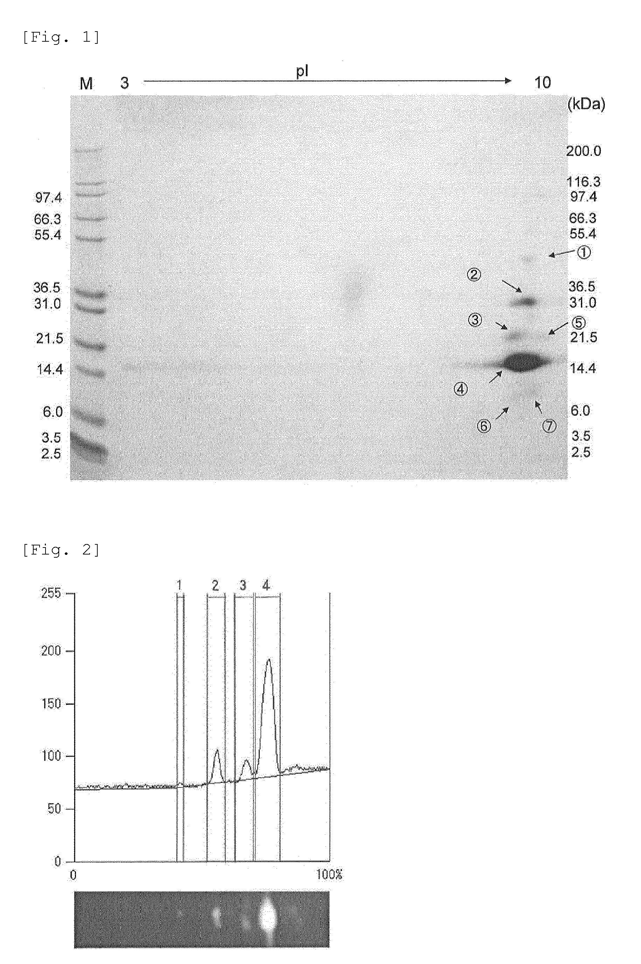

The results of the two-dimensional electrophoresis are shown in Fig. 1. In Fig. 1, the vertical axis indicates molecular weight, and the horizontal axis indicates isoelectric point. Molecular weights for the spots shown in Fig. 1 and types of identified proteins are as shown in Table 1. The circled numbers shown in Fig. 1 with arrows correspond to the numbers mentioned in the column of "Spot No." in Table 1, and the spots [1] to [7] referred to in this specification, respectively.

It became clear that the spot [1] was a protein derived from lactoferrin. The spots [2], [4], [6] and [7] were monomer, dimer, fragment etc. of the protein known as angiogenin-1, as determined by database search and shown in Fig. 6. Further, it became clear that the spot [3] was derived from the protein known as angiogenin-2 as determined by database

search and shown in Fig. 7.

However, type of protein has not been identified for the spot [5].

On the basis of these results, it became clear that the antimicrobial activity enhancing agent of the present invention consists of milk proteins containing the components of the spots [2] to [7] having a molecular weight of 30 kDa or less, and having an isoelectric point of 9.0 to 11.0.

Further, content of substances derived from lactoferrin or lactoferrin hydrolysate in the antimicrobial activity enhancing agent of the present invention was measured. The analysis chart obtained with a densitometer is shown in Fig. 2, and the analysis results are shown in Table 2.

As for the measurement method, among the spots shown in Fig. 1, relative quantification was performed for the lactoferrin-derived substance of the spot [1] as well as substances of the large spots [2], [3] and [4]. Since the peak areas of the other active ingredients of the present invention were extremely small, they were not numerically determined. It became clear that, in Fig. 2, the amount of the lactoferrin-derived substances contained in the antimicrobial activity enhancing agent of the present invention was 1.44 mass % based on the sum of the peak areas of the spots [1] to [4], which was taken as 100.

In addition, since spots other than the spots [1] to [4] were also observed for the antimicrobial activity enhancing agent of the present invention, it is expected that the actual content of components derived from lactoferrin is still lower than 1.44 mass %.

Therefore, the antimicrobial activity enhancing agent of the present invention has a content of components derived from lactoferrin or a lactoferrin hydrolysate among the components is 2.0 mass % or less.

[Table 1]

Table 1

| Spot No. | Molecular weight (kDa) | Identification result |
| --- | --- | --- |
| 1 | 50 | Lactoferrin |
| 2 | 30 | Angiogenin-1 |
| 3 | 17 | Angiogenin-2 |
| 4 | 15 | Angiogenin-1 |
| 5 | 17 | Not determined |
| 6 | 8 to 10 | Angiogenin-1 |
| 7 | 8 to 10 | Angiogenin-1 |

[Table 2]

Table 2

| Peak No. | Integrated value | Ratio (%) | Height |
| --- | --- | --- | --- |
| 1 | 2508 | 1.44 | 5 |
| 2 | 21888 | 12.59 | 33 |
| 3 | 14668 | 8.44 | 19 |
| 4 | 134748 | 77.53 | 112 |
| Total | 173812 | 100.00 | |

[Test Example 2]

This test aimed at confirming that the antimicrobial activity enhancing agent of the present invention enhances the antimicrobial activity of lactoferrin.

(1) Preparation of samples

As lactoferrin, bovine lactoferrin (Morinaga Milk Industry) was diluted with deionized water and used.

As the antimicrobial activity enhancing agent, the antimicrobial activity enhancing agent obtained in Example 1 was diluted with deionized water and used.

Samples 1 to 4 described below were prepared by using lactoferrin and the antimicrobial activity enhancing agent described above.

Sample 1: Bovine lactoferrin (2 mg/mL)
Sample 2: Mixture of bovine lactoferrin (2 mg/mL) and antimicrobial activity enhancing agent (160 $\mu$g/mL). Sample 3: Mixture of bovine lactoferrin (2 mg/mL) and antimicrobial activity enhancing agent (32 $\mu$g/mL). Sample 4: Antimicrobial activity enhancing agent (32 $\mu$g/mL)

(2) Test method

By using 1% Bacto peptone medium, the *Escherichia coli* K-12 strain (NBRC 3301) was inoculated into the medium contained in a polystyrene tube with a 5 ml capacity at a density of $10^6$ CFU/mL, and precultured in an incubator at 37°C for 6 hours to obtain a cell suspension for the test. The precultured *Escherichia coli* was added to a 96-well plate at a cell density of $10^6$ CFU/mL together with each of the aforementioned samples, and cultured at 37°C for 17 hours. After the culture for 17 hours, the culture medium containing *Escherichia coli* was serially diluted with 0.85% physiological saline, and plated on an agar medium (Rapid Media RM-SPC, Morinaga Milk Industry), and culture was performed at 37°C for 16 hours. Then, number of the formed colonies (CFU : Colony Forming Units) was counted to evaluate the antimicrobial activity of each sample.

In addition, *Escherichia coli* was cultured only with the medium as a control, besides the culture using Samples 1 to 4.

(3) Results

The results of Test Example 2 are shown in Table 3. The results of the significant test shown in Table 3 satisfy P < 0.001. Whereas the bacterial count increased to $1.1 \times 10^8$ CFU/mL after 17 hours in the control culture, which was performed only with the medium, the bacterial count decreased from the initial bacterial count to $3.6 \times 10^5$ CFU/mL in the culture using Sample 1 containing lactoferrin.

In the culture using Sample 2 containing lactoferrin and the antimicrobial activity enhancing agent, the bacterial count became $2.5 \times 10^2$ CFU/mL or less, which was significantly lower than that obtained in the culture using lactoferrin. Furthermore, even with Sample 3 in which the amount of the antimicrobial activity enhancing agent was decreased to 1/5, the bacterial count became $2.5 \times 10^2$ CFU/mL or less, and thus the bacterial count decreased to the same extent as that obtained with Sample 2. In addition, on the basis of comparison of the bacterial counts obtained with Samples 1 and 3, it was determined that the antimicrobial activity of Sample 3 was about 1440 times or more as compared with the antimicrobial activity of Sample 1.

With Sample 4 containing only the antimicrobial activity enhancing agent, the bacterial count became $1.1 \times 10^8$ CFU/mL, and it became clear that it does not have antimicrobial activity.
On the basis of the results of this test, it was confirmed that marked increase of the antimicrobial activity was provided by the combination of lactoferrin and the antimicrobial activity enhancing agent of the present invention.

[Table 3]

Table 3

| Sample | Bacterial count (CFU/mL) | S.D. | Survival rate based on control (%) | Change of $Log_{10}$ CFU relative to control | t-Test |
|---|---|---|---|---|---|
| Control | 106, 666, 667 | 5, 773, 503 | - | - | - |
| Sample 1 | 359, 375 | 100, 230 | 0.337 | -2.472 | 0.0000000000 (vs. control) |
| Sample 2 | 250 | 0 | 0.000 | -5.630 | 0.0000374556 (vs. lactoferrin) |
| Sample 3 | 250 | 0 | 0.000 | -5.630 | 0.0000374556 (vs. lactoferrin) |
| Sample 4 | 106, 666, 667 | 7, 637, 626 | 100.000 | 0.000 | 1.0000000000 (vs. control) |

[Test Example 3]

This test aimed at confirming that the antimicrobial activity enhancing agent of the present invention enhances the antimicrobial activity of a lactoferrin hydrolysate.

(1) Preparation of samples

As a lactoferrin hydrolysate, a lactoferrin hydrolysate powder obtained by adjusting the bovine lactoferrin used in Test Example 2 to pH 5 or less, enzymatically digesting it by reaction with 3% pepsin, concentrating the digestion product with a reverse osmotic membrane, and lyophilizing the concentrate was used.
The lactoferrin hydrolysate powder was diluted with deionized water and used.
As the antimicrobial activity enhancing agent, the antimicrobial activity enhancing agent obtained in Example 1 was diluted with deionized water and used.
Samples 5 to 7 described below were prepared by using the aforementioned lactoferrin hydrolysate and antimicrobial activity enhancing agent.

Sample 5: Lactoferrin hydrolysate (400 μg/mL)
Sample 6: Mixture of lactoferrin hydrolysate (400 μg/mL) and antimicrobial activity enhancing agent (32 μg/mL).
Sample 7: Antimicrobial activity enhancing agent (32 μg/mL)

(2) Test method

The test was performed by the same test method as that of Test Example 2. Besides the culture using Samples 5 to 7, *Escherichia coli* was cultured only with the medium as a control.

(3) Results

The results of Test Example 3 are shown in Table 4.
The bacterial count increased to $6.7 \times 10^7$ CFU/mL after 17 hours in the control culture, which was performed only with the medium. In the culture using Sample 5 containing 400 μg/mL of the lactoferrin hydrolysate, the bacterial count decreased to $8.6 \times 10^4$ CFU/mL, but in the culture using Sample 7 further containing the antimicrobial activity enhancing agent, the bacterial count became $2.5 \times 10^2$ CFU/mL or less, and thus marked decrease of the bacterial count was observed compared with the culture using Sample 5. On the basis of comparison of the bacterial counts obtained with Samples 5 and 6, it was determined that the antimicrobial activity of Sample 6 was about 340 times or more as compared with the antimicrobial activity of Sample 5.
With Sample 7 containing only the antimicrobial activity enhancing agent, the bacterial count became $9.0 \times 10^7$ CFU/mL, and thus antimicrobial activity was not observed.
On the basis of the results of this test, it was confirmed that marked increase of the antimicrobial activity was provided by the combination of the lactoferrin hydrolysate and the antimicrobial activity enhancing agent of the present invention.
[Table 4]

Table 4

| Sample | Bacterial count (CFU/mL) | S.D. | Survival rate based on control (%) | Change of $Log_{10}$ CFU relative to control |
|---|---|---|---|---|
| Control | 66, 666, 667 | 2, 886, 751 | - | - |
| Sample 5 | 85, 563 | 22, 738 | 0.128 | -2.892 |
| Sample 6 | 250 | 0 | 0.000 | -5.426 |
| Sample 7 | 90, 000, 000 | 0 | 135.000 | 0.130 |

[Test Example 4]

This test aimed at confirming that the antimicrobial activity enhancing agent of the present invention enhances the antimicrobial activity of the lactoferrin hydrolysate, Lactoferricin (registered trademark).

(1) Preparation of samples

Bovine Lactoferricin powder obtained by hydrolyzing the bovine lactoferrin (Morinaga Milk Industry) used in Test Example 2 with pepsin, purifying the hydrolysate by high performance liquid chromatography, and lyophilizing the purified product was used.
The bovine Lactoferricin powder was diluted with deionized water and used.
As the antimicrobial activity enhancing agent, the antimicrobial activity enhancing agent obtained in Example 1 was diluted with deionized water and used.
Samples 8 to 10 described below were prepared by using the aforementioned bovine Lactoferricin and the antimicrobial activity enhancing agent.

Sample 8: Bovine Lactoferricin (16 $\mu$g/mL)
Sample 9: Mixture of bovine Lactoferricin (16 $\mu$g/mL) and antimicrobial activity enhancing agent (32 $\mu$g/mL) Sample 10: Antimicrobial activity enhancing agent (32 $\mu$g/mL)

(2) Test method

The test was performed by the same test method as that of Test Example 2, except that the culture time was changed to 2 hours. Besides the culture using Samples 8 to 10, *Escherichia coli* was cultured only with the medium as a control.

(3) Results

The results of Test Example 4 are shown in Table 5. The bacterial count increased to 2.9 x $10^6$ CFU/mL in the control culture after 2 hours, in which the culture was performed only with the medium.
In the culture using Sample 8 containing 16 $\mu$g/mL of the bovine Lactoferricin, the bacterial count decreased to 8.8 x $10^3$ CFU/mL, but in the culture using Sample 9 further containing the antimicrobial activity enhancing agent, the bacterial count became 2.5 x $10^2$ CFU/mL or less, and thus marked decrease of the bacterial count was observed.
On the basis of comparison of the bacterial counts obtained with Samples 8 and 9, it was determined that the antimicrobial activity of Sample 9 was 35 times or more as compared with the antimicrobial activity of Sample 8.
With Sample 10 containing only the antimicrobial activity enhancing agent, the bacterial count became 1.4 x $10^6$ CFU/mL, and thus antimicrobial activity was not observed at all.
On the basis of the results of this test, it was confirmed that marked increase of the antimicrobial activity is provided by the combination of the bovine Lactoferricin and the antimicrobial activity enhancing agent of the present invention.
[Table 5]

Table 5

| Sample | Bacterial count (CFU/mL) | S.D. | Survival rate based on control (%) | Change of $Log_{10}$ CFU relative to control |
|---|---|---|---|---|
| Control | 2, 862, 500 | 1,116, 076 | - | - |
| Sample 8 | 8, 833 | 5, 433 | 0.309 | -2.511 |
| Sample 9 | 250 | 0 | 0.009 | -4.059 |
| Sample 10 | 1, 383, 333 | 579, 511 | 48.326 | -0.316 |

[Example 5]

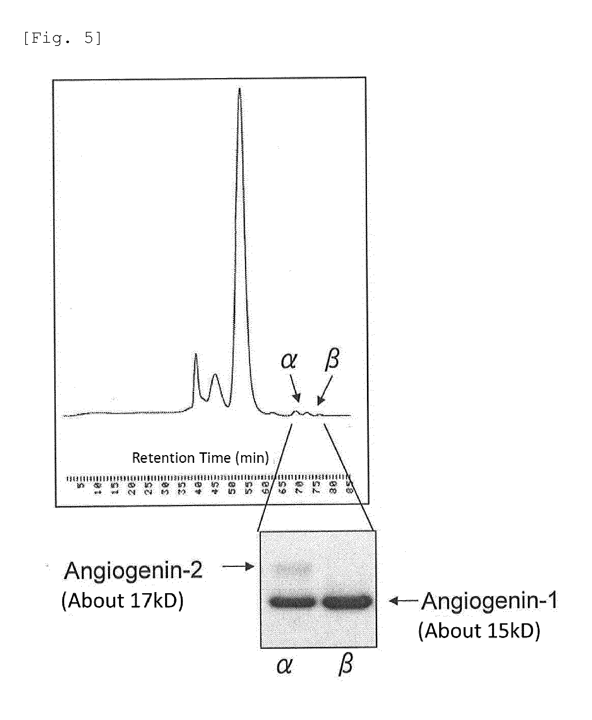

In the same manner as that of Example 1, unsterilized skim milk was flown in a CM-Sephadex C-50 column, the cation exchange resin in the column was sufficiently washed with deionized water, and 0.27 M sodium chloride solution and 1.7 M sodium chloride solution were flown through the column to elute a fraction of proteins adsorbed on the resin.
Further, the eluted protein fraction was dissolved in a citrate buffer (pH 5.2) containing 0.5 M sodium chloride, and the solution was subjected to separation by HPLC using a gel filtration carrier TSKgel G2000SW (TOSOH) to separate two peaks of molecular weights of about 17 kD ($\alpha$) and about 15 kD ($\beta$) shown in Fig. 5. Fig. 5 shows the elution curve observed in the gel filtration HPLC.
Further, the peaks $\alpha$ and $\beta$ were each analyzed by SDS electrophoresis, and it was confirmed that two components of the minor band of a molecular weight of about 17 kDa and the major band of a molecular weight of about 15 kDa were contained in the peak $\alpha$ as shown in Fig. 5, and it was confirmed that one component of the major band of a molecular weight of about 15 kDa was contained in the peak $\beta$.

The bands were excised from the gel, decolorized, reductively alkylated, digested in the gel by trypsinization, and subjected to mass spectrometry using a MALDI-TOF/MS system (trade name: AutoflexII, BRUKER), and the proteins were identified by searching the database of NCBI (National Center for Biotechnology Information, http://www.ncbi.nlm.nih.gov/) using the MASCOT software produced by Matrix Science. As a result, it was found that the component of about 15 kDa contained in both of the peaks $\alpha$ and $\beta$ was angiogenin-1, and the component of about 17 kDa contained in the peak $\alpha$ was angiogenin-2.

[Test Example 5]

This test was performed in order to confirm that angiogenin-1 and angiogenin-2 has an action of enhancing the antimicrobial activity of lactoferrin.

(1) Preparation of samples

As lactoferrin, bovine lactoferrin (Morinaga Milk Industry) was diluted with deionized water and used. Angiogenin-1 and angiogenin-2 separated by the method described in Example 5 were diluted with deionized water and used.

Samples 11 to 15 described below were prepared by using the aforementioned bovine lactoferrin, and separated angiogenin-1 and angiogenin-2.

Sample 11: Bovine lactoferrin (2 mg/mL)
Sample 12: Mixture of bovine lactoferrin (2 mg/mL) and angiogenin-1 (32 $\mu$g/mL)
Sample 13: Mixture of bovine lactoferrin (2 mg/mL) and mixture of angiogenin-1 and angiogenin-2 (32 $\mu$g/mL each)
Sample 14: Angiogenin-1 (32 $\mu$g/mL)
Sample 15: Mixture of angiogenin-1 and angiogenin-2 (32 $\mu$g/mL each)

By using 1% Bacto peptone medium (Becton Dickinson), the *Escherichia coli* K-12 strain (NBRC 3301) was inoculated to the medium contained in a polystyrene tube with a 5 ml capacity at a density of $10^6$ CFU/mL, and precultured in an incubator at 37°C for 6 hours to obtain a cell suspension for the test.

(2) Test method

The cell suspension for the test was added to a 96-well plate at a cell density of $10^6$ CFU/mL together with each of the aforementioned samples, and culture was performed at 37°C for 17 hours.

After the culture for 17 hours, the culture medium containing *Escherichia coli* was serially diluted with 0.85% physiological saline, and plated on an agar medium (Rapid Media RM-SPC, Morinaga Milk Industry), and then culture was performed at 37°C for 16 hours. Then, number of the formed colonies (CFU : Colony Forming Unit) was measured to evaluate the antimicrobial activity of each sample.

In addition, *Escherichia coli* was cultured only with the medium as a control, besides the culture using Samples 11 to 15.

(3) Test results

The results of this test are shown in Table 6.

As a result, whereas the bacterial count increased to $1.3 \times 10^8$ CFU/mL after 17 hours in the control culture, which was performed only with the medium, the bacterial count decreased from the initial bacterial count to $2.9 \times 10^5$ CFU/mL in the culture using Sample 11 containing lactoferrin.

On the other hand, in the culture using Samples 12 and 13 containing lactoferrin and angiogenin-1 or angiogenin-1 and angiogenin-2, the bacterial count became lower than $2.5 \times 10^4$ CFU/mL and about $4.1 \times 10^4$ CFU/mL, respectively, and thus the bacterial count markedly decreased compared with that obtained with lactoferrin.

Based on the comparison of the bacterial count of the sample 11 and the sample 12, it was determined that the antibacterial activity of the sample 12 against the sample 11 was about 12 times or more. Moreover, based on the comparison of the bacterial count of the sample 11 and the sample 13, it was also determined that the antibacterial activity of the sample 13 against the sample 11 was about 7 times or more.

With Samples 14 and 15 containing only angiogenin-1, or both angiogenin-1 and angiogenin-2, the bacterial count became about $1.9 \times 10^8$ CFU/mL and $1.5 \times 10^8$ CFU/mL, respectively, and it became clear that angiogenin-1 and angiogenin-2 themselves do not have antimicrobial activity.

[Table 6]

Table 6

| Sample | Bacterial count (CFU/mL) | S.D. | Survival rate based on control (%) | Change of $Log_{10}$ CFU relative to control | t-Test |
|---|---|---|---|---|---|
| Control | 132, 500, 000 | 25, 248, 762 | - | - | - |
| Sample 11 | 292,143 | 93, 713 | 0.220 | -2.657 | 0.0000000242 (vs. control) |
| Sample 12 | 24, 650 | 7, 857 | 0.019 | -3.730 | 0.0000001678 (vs. lactoferrin) |
| Sample 13 | 41, 295 | 13, 641 | 0.031 | -3.506 | 0.0000001385 (vs. lactoferrin) |
| Sample 14 | 191, 666, 667 | 74, 408, 781 | 144.654 | 0.160 | 0.0949066006 (vs. control) |
| Sample 15 | 150, 000, 000 | 54, 863, 467 | 113.208 | 0.054 | 0.4940658661 (vs. control) |

[Test Example 6]

This test was performed in order to confirm that the antimicrobial activity enhancing agent of the present invention has an action of enhancing the antimicrobial activity of lactoferrin against various bacteria

(1) Preparation of samples

As lactoferrin, bovine lactoferrin (Morinaga Milk Industry) was diluted with deionized water and used. Further, an antimicrobial activity enhancing agent prepared by the method described in Example 1 (containing angiogenin-1 and angiogenin-2) was diluted with deionized water and used.

Samples 16 to 22 described below were prepared by using the aforementioned bovine lactoferrin.

Sample 16: Bovine lactoferrin (8 mg/mL)
Sample 17: Bovine lactoferrin (4 mg/mL)
Sample 18: Bovine lactoferrin (2 mg/mL)
Sample 19: Bovine lactoferrin (1 mg/mL)
Sample 20: Bovine lactoferrin (0.5 mg/mL)
Sample 21: Bovine lactoferrin (0.25 mg/mL)
Sample 22: Bovine lactoferrin (0.125 mg/mL)

Further, Samples 23 to 29 corresponding to Samples 16 to 22 further containing the antimicrobial activity enhancing agent (32 $\mu$g/mL) were prepared.

Furthermore, Sample 30 containing only the antimicrobial activity enhancing agent (32 $\mu$g/mL) was also prepared.

By using 1% Bacto peptone medium (Becton Dickinson), each of *Escherichia coli* 0111, *Cronobacter sakazaki* ATCC 12868, *Salmonella enteritidis* IID 604, *Klebsiella pneumoniae* JCM 1662T, *Pseudomonas aeruginosa* MMI 603, *Staphylococcus aureus* JCM 2151 and *Staphylococcus epidermidis* JCM 2414T was inoculated to the medium contained in a polystyrene tube with a 5 ml capacity at a density of $10^6$ cells/mL, and precultured in an incubator at 37°C for 6 hours to obtain a cell suspension for the test.

In a similar manner, by using the PYG medium (1% Bacto peptone, 0.05% yeast extract (Becton Dickinson), 1% glucose (Wako Pure Chemical Industries)), *Streptococcus mutans* JCM 5705T was inoculated to the medium contained in a polystyrene tube with a 5 ml capacity at a density of $10^6$ cells/mL, and precultured in an incubator at 37°C for 6 hours to obtain a cell suspension for the test. Further, by using the Sabouraud medium (1% Bacto peptone, 2% glucose), *Candida albicans* TIMM1768 was inoculated to the medium contained in a polystyrene tube with a 5 ml capacity at a density of $10^6$ cells/mL, and precultured in an incubator at 37°C for 6 hours to obtain a cell suspension for the test.

(2) Test method

Each of the cell suspensions for the test was added to a 96-well plate at a cell density of $10^6$ cells/mL together with each of the aforementioned samples, and culture was performed at 37°C for 17 hours. After the culture for 17 hours, absorbance of the medium was measured at 630 nm using a microplate reader (Corona Electric). In addition, each bacterium was cultured only with the medium as a control, besides the culture using Samples 16 to 30. The minimum lactoferrin concentration that significantly suppressed growth of bacterium and provided an absorbance corresponding to 50% of the absorbance observed for the control or less was considered as the minimum inhibitory concentration (MIC), and on the basis of comparison of MICs obtained with lactoferrin containing the antimicrobial activity enhancing agent and lactoferrin not containing the antimicrobial activity enhancing agent, the action of the antimicrobial activity enhancing agent for enhancing suppression of growth of the various bacteria was evaluated.

(3) Test results

The results of this test are shown in Table 7. In Table 7, LF represents lactoferrin, and MIC represents minimum inhibitory concentration.
As a result, in the case of *Escherichia coli* 0111, when lactoferrin was added (Samples 16 to 22), MIC was 2 mg/mL. On the other hand, when 32 μg/mL of the antimicrobial activity enhancing agent was further added to lactoferrin (Samples 23 to 29), MIC became 1 mg/mL, and thus decrease of MIC was observed as compared with the cases using only lactoferrin.
With the antimicrobial activity enhancing agent alone (Sample 30), suppression of growth of the bacteria was not observed at all, and MIC of the antimicrobial activity enhancing agent was 32 μg/mL or more.
Similarly, also in the cases of *Cronobacter sakazaki* ATCC 12868, *Salmonella enteritidis* IID 604, *Klebsiella pneumoniae* JCM 1662T, *Pseudomonas aeruginosa* MMI 603, *Staphylococcus aureus* JCM 2151, *Staphylococcus epidermidis* JCM 2414T, *Streptococcus mutans* JCM 5705T and *Candida albicans* TIMM1768, when the results obtained with samples containing lactoferrin alone (Samples 16 to 22) and Samples containing lactoferrin and 32 μg/mL of the antimicrobial activity enhancing agent (Samples 23 to 29) were compared, distinct decrease of MIC was observed for the latter. In addition, for all of the Gram-negative bacteria, Gram-positive bacteria and fungus, addition of the antimicrobial activity enhancing agent to lactoferrin decreased MIC to 1/2 or less of that observed by using lactoferrin alone. Further, with the sample comprising the antimicrobial activity enhancing agent alone (Sample 30), the growth inhibition effect was not observed for all the bacteria as in the case of *Escherichia coli* O111.
On the basis of the results of this test, it became clear that by preparing a composition comprising an antimicrobial substance such as lactoferrin and the antimicrobial activity enhancing agent of the present invention, a composition for antimicrobial use in which the activity of the conventional antimicrobial substance was markedly enhanced for a wide range of bacteria could be provided.
[Table 7]

Table 7

| | | MIC (μg/mL) | MIC (mg/mL) | |
| | Test strain | Antimicrobial activity enhancing agent | LF | LF (containing 32 μg/mL of antimicrobial activity enhancing agent) |
|---|---|---|---|---|
| Gram-negative bacteria | *Escherichia coli* 0111 | >32 | 2 | 1 |
| | *Cronobacter sakazaki* ATCC 12868 | >32 | 8 | 1 |
| | *Salmonella enteritidis* IID 604 | >32 | 4 | 1 |
| | *Klebsiella pneumoniae* JCM 1662T | >32 | 8 | 0.5 |
| | *Pseudomonas aeruginosa* MMI 603 | >32 | 8 | 1 |

(continued)

| | Test strain | MIC (μg/mL) | MIC (mg/mL) | |
| --- | --- | --- | --- | --- |
| | | Antimicrobial activity enhancing agent | LF | LF (containing 32 μg/mL of antimicrobial activity enhancing agent) |
| Gram-positive bacteria | *Staphylococcus aureus* JCM 2151 | >32 | 0.5 | 0.125 |
| | *Streptococcus mutans* JCM 5705T | >32 | >8 | 0.125 |
| | *Staphylococcus epidermidis* JCM 2414T | >32 | 1 | 0.125 |
| Fungus | *Candida albicans* TIMM1768 | >32 | 1 | 0.125 |

[Test Example 7]

This test was performed in order to confirm that the antimicrobial activity enhancing agent of the present invention has an action of enhancing the antimicrobial activity of Lactoferricin against various bacteria.

(1) Preparation of samples

As Lactoferricin, Lactoferricin powder obtained by hydrolyzing bovine lactoferrin (Morinaga Milk Industry) with pepsin, purifying the hydrolysate by high performance liquid chromatography, and lyophilizing the purified product was used. The Lactoferricin powder was diluted with deionized water and used. As the antimicrobial activity enhancing agent, an antimicrobial activity enhancing agent prepared by the method described in Example 1 (containing angiogenin-1 and angiogenin-2) was diluted with deionized water and used.

Samples 31 to 35 described below were prepared by using the aforementioned bovine Lactoferricin.

Sample 31: Bovine Lactoferricin (32 μg/mL)
Sample 32: Bovine Lactoferricin (16 μg/mL)
Sample 33: Bovine Lactoferricin (8 μg/mL)
Sample 34: Bovine Lactoferricin (4 μg/mL)
Sample 35: Bovine Lactoferricin (2 μg/mL)

Further, Samples 36 to 40 corresponding to Samples 31 to 35 further containing the antimicrobial activity enhancing agent (32 μg/mL) were prepared.

Furthermore, Sample 41 containing only the antimicrobial activity enhancing agent (32 μg/mL) was also prepared.

By using 1% Bacto peptone medium (Becton Dickinson), each of *Escherichia coli* 0111, *Cronobacter sakazaki* ATCC 12868, *Klebsiella pneumoniae* JCM 1662T, *Pseudomonas aeruginosa* MMI 603, and *Staphylococcus aureus* JCM 2151 was inoculated to the medium contained in a polystyrene tube with a 5 ml capacity at a density of $10^6$ cells/mL, and precultured in an incubator at 37°C for 6 hours to obtain a cell suspension for the test. In a similar manner, by using the PYG medium (1% Bacto peptone, 0.05% yeast extract (Becton Dickinson), 1% glucose (Wako Pure Chemical Industries)), *Streptococcus mutans* JCM 5705T was inoculated to the medium contained in a polystyrene tube with a 5 ml capacity at a density of $10^6$ cells/mL, and precultured in an incubator at 37°C for 6 hours to obtain a cell suspension for the test. Further, by using the Sabouraud medium (1% Bacto peptone, 2% glucose), *Candida albicans* TIMM1768 was inoculated to the medium contained in a polystyrene tube with a 5 ml capacity at a density of $10^6$ cells/mL, and precultured in an incubator at 37°C for 6 hours to obtain a cell suspension for the test.

(2) Test method

Each of the cell suspensions for the test was added to a 96-well plate at a cell density of $10^6$ cells/mL together with each of the aforementioned samples, and culture was performed at 37°C for 17 hours.

After the culture for 17 hours, absorbance of the medium was measured at 630 nm using a microplate reader (Corona Electric). In addition, each bacterium was cultured only with the medium as a control, besides the culture using Samples 31 to 41. The minimum Lactoferrin concentration that significantly suppressed growth of bacterium and provided an absorbance corresponding to 50% of the absorbance observed for the control or lower was considered as the minimum inhibitory concentration (MIC), and on the basis of comparison of MICs obtained with Lactoferrin containing the antimicrobial activity enhancing agent and Lactoferrin not containing the antimicrobial activity enhancing agent, the action of the antimicrobial activity enhancing agent for enhancing suppression of growth of the various bacteria was evaluated.

(3) Test results

The results of this test are shown in Table 8. In Table 8, LFcin represents Lactoferrin, and MIC represents minimum inhibitory concentration.

As a result, in the case of *Escherichia coli* 0111, when Lactoferricin was added (Samples 31 to 35), MIC was 8 μg/mL. On the other hand, when the antimicrobial activity enhancing agent (32 μg/mL) was further added to Lactoferricin (Samples 36 to 40), MIC became 2 μg/mL, and thus decrease of MIC was observed as compared with the cases using Lactoferrin alone.

With the antimicrobial activity enhancing agent alone (Sample 41), suppression of growth of the bacterium was not observed at all, and MIC of the antimicrobial activity enhancing agent was 32 μg/mL or more.

Similarly, also in the cases of *Cronobacter sakazaki* ATCC 12868, *Klebsiella pneumoniae* JCM 1662T, *Pseudomonas aeruginosa* MMI 603, *Staphylococcus aureus* JCM 2151, *Streptococcus mutans* JCM 5705T and *Candida albicans* TIMM 1768, when the results obtained with samples containing Lactoferricin alone (Samples 31 to 35) and Samples containing Lactoferricin and 32 μg/mL of the antimicrobial activity enhancing agent (Samples 36 to 40), distinct decrease of MIC was observed for the latter. In addition, for all of the Gram-negative bacteria, Gram-positive bacteria and fungus, addition of the antimicrobial activity enhancing agent to Lactoferricin decreased MIC to 1/2 or less of that observed by using Lactoferrin alone.

Further, with the sample comprising the antimicrobial activity enhancing agent alone (Sample 41), the growth inhibition effect for the bacteria was not observed for all the bacteria as in the case of *Escherichia coli* 0111.

On the basis of the results of this test, it became clear that by preparing a composition comprising an antimicrobial substance such as Lactoferricin and the antimicrobial activity enhancing agent of the present invention, a composition for antimicrobial use in which the activity of the conventional antimicrobial substance was markedly enhanced for a wide range of bacteria could be provided.

[Table 8]

Table 8

| | | MIC (μg/mL) | | |
|---|---|---|---|---|
| | Test strain | Antimicrobial activity enhancing agent | LFcin | LFcin (containing 32 μg/mL of antimicrobial activity enhancing agent) |
| Gram-negative bacteria | *Escherichia coli* 0111 | >32 | 8 | 4 |
| | *Cronobacter sakazaki* ATCC 12868 | >32 | 32 | 16 |
| | *Klebsiella pneumoniae* JCM 1662T | >32 | 32 | 8 |
| | *Pseudomonas aeruginosa* MMI 603 | >32 | 32 | 16 |
| Gram-positive bacteria | *Staphylococcus aureus* JCM 2151 | >32 | 8 | 4 |
| | *Streptococcus mutans* JCM 5705T | >32 | 32 | 8 |
| Fungus | *Candida albicans* TIMM 1768 | >32 | >32 | 32 |

Industrial Applicability

By adding the antimicrobial activity enhancing agent of the present invention to an antimicrobial agent containing lactoferrin or a lactoferrin hydrolysate as an active ingredient, the antimicrobial activity of lactoferrin or the lactoferrin hydrolysate can be markedly improved.

Further, since the antimicrobial activity enhancing agent of the present invention exhibits the effect even when it is mixed only in an extremely small amount with lactoferrin or a lactoferrin hydrolysate, it can be blended with foods, drinks, feeds, drugs, cosmetics, and so forth without affecting composition and properties of the products.

Furthermore, since the antimicrobial activity enhancing agent of the present invention is obtained from components derived from milk, it scarcely has a risk of side reaction, and it can be taken in daily basis with feeling safe.

## Claims

1. An antimicrobial activity enhancing agent for lactoferrin or a lactoferrin hydrolysate, which contains a milk protein as an active ingredient and has the following properties:

(A) an action of enhancing the antimicrobial activity of lactoferrin or a lactoferrin hydrolysate,
(B) a molecular weight of 30 kDa or less,
(C) an isoelectric point of 9.0 to 11.0.

2. The antimicrobial activity enhancing agent according to claim 1, wherein the milk protein contains lactoferrin or a lactoferrin hydrolysate in an amount of 2.0 mass % or less.

3. The antimicrobial activity enhancing agent according to claim 1 or 2, the milk protein is angiogenin-1 and/or angiogenin-2.

4. The antimicrobial activity enhancing agent according to any one of claims 1 to 3, wherein the milk protein per se does not have antimicrobial activity against *Escherichia* coli.

5. The antimicrobial activity enhancing agent according to any one of claims 1 to 4, wherein the milk protein has a property that, when the protein is used together with lactoferrin or a lactoferrin hydrolysate, it enhances the antimicrobial activity to 7 times or more of lactoferrin or the lactoferrin hydrolysate against *Escherichia coli.*

6. An antimicrobial composition comprising the antimicrobial activity enhancing agent according to any one of claims 1 to 5, and one or plural kinds of substances selected from the group consisting of lactoferrin and a lactoferrin hydrolysate, wherein mass ratio of the milk protein in the antimicrobial activity enhancing agent to lactoferrin and a lactoferrin hydrolysate is 1/80 or more.

7. A drug for antimicrobial use containing the antimicrobial composition according to claim 6.

8. A food or drink containing the antimicrobial composition according to claim 6.

9. A feed containing the antimicrobial composition according to claim 6.

10. Use of a milk protein having the following properties for the production of an antimicrobial activity enhancing agent for lactoferrin or a lactoferrin hydrolysate:

    (A) an action of enhancing the antimicrobial activity of lactoferrin or a lactoferrin hydrolysate,
    (B) a molecular weight of 30 kDa or less,
    (C) an isoelectric point of 9.0 to 11.0.

11. The use according to claim 10, wherein the milk protein contains lactoferrin or a lactoferrin hydrolysate in an amount of 2.0 mass % or less.

12. The use according to claim 10 or 11, wherein the milk protein is angiogenin-1 and/or angiogenin-2.

13. The use according to any one of claims 10 to 12, wherein the milk protein in itself does not have antimicrobial activity against *Escherichia coli.*

14. The use according to any one of claims 10 to 13, wherein the milk protein has a property that, when the protein is used together with lactoferrin or a lactoferrin hydrolysate, it enhances the antimicrobial activity to 7 times or more of lactoferrin or the lactoferrin hydrolysate against *Escherichia coli.*

15. A method for producing an antimicrobial activity enhancing agent for lactoferrin or a lactoferrin hydrolysate comprising a milk protein, which comprises:

    (a) the step of contacting raw milk with a cation exchange resin to adsorb proteins in the raw milk to the cation exchange resin,
    (b) the step of washing the cation exchange resin having undergone the adsorption treatment, and flowing an elution solvent to elute a protein fraction having an isoelectric point of 9.0 to 11.0, and
    (c) the step of subjecting the eluted protein fraction to membrane filtration using an ultrafiltration membrane having a molecular weight cutoff of 30 kDa to prepare the milk protein having the following properties:

        (A) an action of enhancing the antimicrobial activity of lactoferrin or a lactoferrin hydrolysate,

(B) a molecular weight of 30 kDa or less,
(C) an isoelectric point of 9.0 to 11.0.

[Fig. 1]

[Fig. 2]

[Fig. 3]

[Fig. 4]

[Fig. 5]

[Fig. 6]

Mascot Search Results   No. 1 (Sequence Coverage:70%)

AQDDYRYIHF LTQHYDAKPK GRNDEYCFNM MKNRRLTRPC KDRNTFIHGN
KNDIKAICED RNGQPYRGDL RISKSEFQIT ICKHKGGSSR PPCRYGATED
SRVIVVGCEN GLPVHFDESF ITPRH


Mascot Search Results   No. 2 (Sequence Coverage:59%)

MVMVLSPLLL VFILGLGLTP VAPAQDDYRY IHFLTQHYDA KPKGRNDEYC
FNMMKNRRLT RPCKDRNTFI HGNKNDIKAI CEDRNGQPYR GDLRISKSEF
QITICKHKGG SSRPPCRYGA TEDSRVIVVG CENGLPVHFD ESFITPRH


Mascot Search Results   No. 3 (Sequence Coverage:59%)

MVMVLSPLFL VFILGLGLTP VAPAQDDYRY IHFLTQHYDA KPKGRNDEYC
FNMMKNRRLT RPCKDRNTFI HGNKNDIKAI CEDRNGQPYR GDLRISKSEF
QITICKHKGG SSRPPCRYGA TEDSRVIVVG CENGLPVHFD ESFITPRH

[Fig. 7]

Mascot Search Results   No. 4 (Sequence Coverage:47%)

QNDAYRGFLR KHYDPSPTGH DDRYCNTMME RRNMTRPCKD TNTFIHGNSD
DIRAVCDDRN GEPYRNGLRR SRSPFQVTTC RHRGGSPRPP CRYRAFRANR
VIVIRCRDGF PIHLEENFIP PRP


Mascot Search Results   No. 5 (Sequence Coverage:39%)

MVMVLSPLFL VFMLDLGLTP QTLAQNDAYR GFLRKHYDPS PTGHDDRYCN
TMMERRNMTR PCKDTNTFIH GNSDDIRAVC DDRNGEPYRN GLRRSRSPFQ
VTTCRHRGGS PRPPCRYRAF RANRVIVIRC RDGFPIHLEE NFIPPRP

**INTERNATIONAL SEARCH REPORT**

| | International application No. |
|---|---|
| | PCT/JP2011/067488 |

A. CLASSIFICATION OF SUBJECT MATTER
*A61K38/00*(2006.01)i, *A23K1/16*(2006.01)i, *A23L1/305*(2006.01)i, *A23L2/52*
(2006.01)i, *A61K38/22*(2006.01)i, *A61P31/04*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K38/00, A23K1/16, A23L1/305, A23L2/52, A61K38/22, A61P31/04, A61P43/00

Documentation searched other than minimum documentation to the extent that such documents are included in the fields searched
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| X/Y | INTERNATIONAL DAIRY JOURNAL, 2010.06, VOL.10, NO.6, P.400-407 | 1-7,10-14/8, 9,15 |
| X/Y | WO 2007/142542 A2 (DEC INTERNATIONAL NZ LTD.), 13 December 2007 (13.12.2007), entire text & US 2011/0008361 A & EP 2040735 A & NZ 547859 A & CA 2687890 A | 6,7/1-5,8-15 |
| Y | JP 2006-6257 A (Yugen Kaisha Food Design Kenko Kenkyusho), 12 January 2006 (12.01.2006), entire text (Family: none) | 8,9 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 23 August, 2011 (23.08.11) | 30 August, 2011 (30.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/067488

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2006-187300 A  (Morinaga Milk Industry Co., Ltd.), 20 July 2006 (20.07.2006), entire text (Family: none) | 8,9 |
| Y | JP 7-48274 A  (Immuno Japan Inc.), 21 February 1995 (21.02.1995), entire text (Family: none) | 7,9 |
| Y | JP 6-312922 A  (Snow Brand Milk Products Co., Ltd.), 08 November 1994 (08.11.1994), paragraphs [0002], [0008] to [0009] & US 5698185 A           & EP 622071 A1 & DE 69411073 C | 15 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11292789 A
- JP 5255109 A
- JP 5320067 A
- JP 6199698 A
- JP 9124504 A
- JP 11092375 A
- JP 3171736 A

**Non-patent literature cited in the description**

- **WELSH, J.K. et al.** *Journal of Pediatrics,* 1979, vol. 94, 1-9
- **NONNECKE, B.J. et al.** *Journal of Dairy Science,* 1984, vol. 67, 606-613
- **BELLAMY, W. et al.** *Biochimica et Biophysica Acta,* 1992, vol. 1121, 130-136
- **BELLAMY, W. et al.** *Journal of Applied Bacteriology,* 1992, vol. 73, 472-479
- **HOOPER, L.V. et al.** *Nature Immunology,* 2003, vol. 4, 269-273